# FASCICULE DE BREVET EUROPEEN

(11) **EP 1 631 586 B1**
(45) Date de publication et mention de la délivrance du brevet: **22.08.2007**
(21) Numéro de dépôt: 04767332.2
(22) Date de dépôt: 11.06.2004
(51) Int. Cl.: C07K 14/47

(54) **ANALOGUES PEPTIDIQUES COMPRENANT AU MOINS UN RESIDU AZA-BETA 3 AMINOACYLE, ET LEURS UTILISATIONS, NOTAMMENT EN THERAPIE**
PEPTIDANALOGA, DIE WENIGSTENS EINEN AMINOACY-AZA-BETA-3-REST ENTHALTEN, UND DEREN VERWENDUNG, INSBESONDERE FÜR DIE THERAPIE
PEPTIDE ANALOGUES COMPRISING AT LEAST ONE TYPE OF AMINOACYL AZA-BETA 3 AND THE USE THEREOF, IN PARTICULAR FOR THERAPY

(30) Priorité: 11.06.2003 FR 0306992
(43) Date de publication de la demande: 08.03.2006
(73) Titulaire: CENTRE NATIONAL DE LA RECHERCHE SCIENTIFIQUE (CNRS), 75794 Paris Cedex 16 (FR)
(72) Inventeur: BAUDY FLOC'H, Michèle, F-35000 Rennes (FR); BUSNEL, Olivier, F-50140 Fontenay (FR); MULLER, Sylviane, F-67000 Strasbourg (FR)
(74) Mandataire: Grosset-Fournier, Chantal Catherine
(86) Numéro de dépôt international: PCT/FR2004/001467
(87) Numéro de publication internationale: WO 2004/111086

(56) Documents cités:
- CA-A- 2 097 533
- FR-A- 2 828 884
- US-A1- 2003 021 797
- DECKER P ET AL.: "Identification of a Minimal T Cell Epitope Recognized by Antinucleosome Th Cells in the C-Terminal Region of Histone H4" THE JOURNAL OF IMMUNOLOGY, vol. 165, no. 2, 2000, pages 654-662, XP002277566
- CHEGUILLAUME ARNAUD ET AL: "Solution synthesis and characterization of aza-.beta.3-peptides (N.alpha.-substituted hydrazino acetic acid oligomers)" JOURNAL OF ORGANIC CHEMISTRY, AMERICAN CHEMICAL SOCIETY. EASTON, US, vol. 66, 2001, pages 4923-4929, XP002197673 ISSN: 0022-3263
- BOUGET K ET AL: "HYDRAZINO-AZA AND N-AZAPEPTOIDS WITH THERAPEUTIC POTENTIAL AS ANTICANCER AGENTS" BIOORGANIC & MEDICINAL CHEMISTRY, ELSEVIER SCIENCE LTD, GB, vol. 11, no. 23, 17 November 2003 (2003-11-17), pages 4881-4889, XP001187599 ISSN: 0968-0896

## Description

La présente invention a pour objet des analogues de peptides ou protéines parents, ces analogues peptidiques, comprenant au moins un résidu aza-β³ aminoacyle, ainsi que leurs utilisations dans des compositions pharmaceutiques ou pour le diagnostic de pathologies dans lesquelles sont impliqués les peptides ou protéines parents susmentionnés.

L'identification des régions antigéniques (ou épitopes) reconnues par les cellules T et la compréhension des bases moléculaires et cellulaires de la reconnaissance antigéniques sont considérées comme des étapes clés dans la conception et le développement de stratégies vaccinales et d'immunomodulation. L'immunisation à l'aide de peptides correspondant à des épitopes du non-soi (par exemple viraux, bactériens) ou du soi (par exemple tumoraux) pour induire des anticorps et/ou des lymphocytes T auxiliaires (Th) ou des lymphocytes cytotoxiques (CTL) spécifiques de la tumeur ou du virus, est aujourd'hui une stratégie particulièrement prometteuse dans le développement de vaccins synthétiques. Dans le cas des vaccins antiviraux par exemple, les peptides présentent plusieurs avantages majeurs sur les préparations traditionnelles de virus atténués ou inactivés, à savoir une production plus simple, chimiquement définie et parfaitement contrôlable, ainsi qu'une meilleure stabilité à température ambiante. Des approches thérapeutiques basées sur des épitopes T CD4⁺ d'antigènes du soi sont aussi proposées.

En pratique, cependant, les peptides se révèlent souvent peu immunogènes et ne permettent pas d'obtenir des titres élevés d'anticorps capables de réagir avec la protéine native ou la particule virale. Ces limitations à l'utilisation des peptides dans le développement de vaccins synthétiques sont probablement liées à une biodégradabilité importante dans les fluides biologiques, une mauvaise diffusion à travers les systèmes membranaires et par le manque de sélectivité vis-à-vis de la cible.

Différentes approches ont été développées pour "transformer" les peptides en molécules capables d'induire une réponse immune humorale ou cellulaire plus forte et plus spécifique. L'introduction dans des peptides antigéniques de liaisons pseudopeptidiques est une stratégie des plus intéressantes pour améliorer leurs caractéristiques physico-chimiques propres et leur aptitude à interagir avec les effecteurs du système immunitaire. Malgré les applications potentielles importantes dans le domaine du diagnostic, de la vaccination ou de l'immunomodulation, et l'étendue des connaissances de ces analogues acquises dans des domaines chimique et pharmacologique, les pseudopeptides sont encore peu utilisés en immunologie.

L'article du Decker et al., The Journal of Immunology, 2000, 165: 654-662 concerne l'étude de l'histone H4 du nucléosome et l'identification d'un épitope particulier de celle-ci, l'épitope H4 (88-99). Cet article décrit également l'utilisation de cet épitope dans le cadre de la prévention ou du traitement du lupus érythémateux disséminé.

Le document FR 2 828 884 concerne l'utilisation des hydrazinopeptoides de formule (I) suivante: 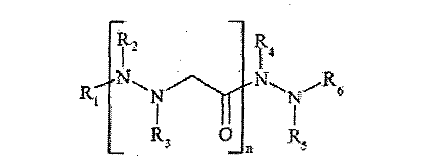 pour la préparation d'un médicament destiné notamment au traitement des cancers.

Dans ce document, R₂ et R₃ peuvent représenter un atome d'hydrogène ou un groupe alkyle de 1 à 10 atomes de carbone, éventuellement substitué.

La présente invention a pour but de fournir des analogues peptidiques résistants aux enzymes de dégradation, et capables de mimer l'activité de divers peptides natifs, agents de vaccination ou d'immunomodulation.

L'invention a plus particulièrement pour but de fournir des analogues peptidiques qui se caractérisent par l'introduction de monomères, ne présentant pas de centres chiraux carbonés, ce qui permet de s'affranchir des difficultés liées à la synthèse asymétrique et aux problèmes d'épimérisation. Cette famille d'analogues peptidiques constitue une nouvelle classe de peptidomimétiques, dans lesquels les résidus (chaînes latérales) sont portés par des atomes d'azote chiraux à configuration non fixée, ce qui leur confère une grande adaptabilité spatiale. Le positionnement correct des peptidomimétiques construits selon ce principe, dans un site enzymatique se produit à la fois par le déplacement d'équilibres conformationnel et configurationnel. L'action d'un tel composé, d'un point de vue stéréochimique, est équivalente à celle d'un mélange de diastéréoisomères en équilibre rapide, l'interaction avec le site enzymatique déplaçant l'équilibre vers le ou les stéréoisomères les plus affins. D'autres bénéfices potentiels peuvent également en résulter, tels que, d'un point de vue chimique, une simplification des méthodes de synthèse (suppression des problèmes stéréochimiques), et d'autre part, une plus grande résistance de tels analogues aux squelettes modifiés, vis-à-vis de l'action des peptidases. La synthèse préalable de ces monomères, autorise l'introduction d'une bonne diversité de chaînes latérales, aussi bien en série protéogénique que non protéogénique, et donc permet de moduler dans une certaine mesure, leur affinité et leur lipophilie.

L'invention a également pour but de fournir des compositions pharmaceutiques comprenant de tels analogues peptidiques, ainsi que des méthodes de diagnostic *in vitro* de pathologies impliquant les peptides parents dont sont issus ces analogues peptidiques, et des kits pour la mise en oeuvre de ces méthodes.

La présente invention a pour objet l'utilisation d'analogues de peptides ou protéines parents, ces analogues peptidiques, encore désignés peptides hybrides, comprenant au moins un résidu aza-β³-aminoacyle, à savoir :
- un résidu répondant à la formule (A) suivante lorsqu'il est situé en position N-terminale, 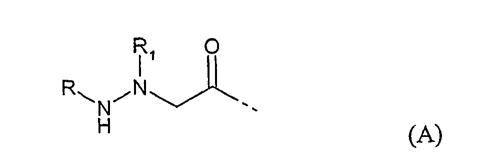 dans laquelle R représente H ou un groupe protecteur de la fonction amine des aminoacides, tels que Fmoc (fluorénylméthyloxycarbonyle), Boc (tertio-butyloxycarbonyle), ou Z (benzyloxycarbonyle), et R₁ représente une chaîne latérale choisie parmi celles des aminoacides,
- un résidu répondant à la formule (B) suivante lorsqu'il est situé en position C-terminale, 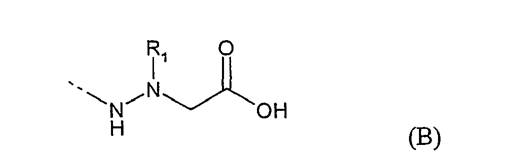 dans laquelle R₁ représente une chaîne latérale choisie parmi celles des aminoacides,
- un résidu répondant à la formule (C) suivante lorsqu'il est situé dans la chaîne desdits peptides hybrides, 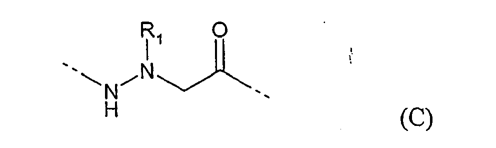 dans laquelle R₁ représente une chaîne latérale choisie parmi celles des aminoacides, lesdits peptides hybrides étant caractérisés en ce qu'ils sont issus de l'épitope 88-99 de l'histone H4 à titre de peptide parent, correspondant à SEQ ID NO : 1, dont l'un au moins des aminoacides initiaux est substitué par un résidu analogue aza-β³ aminoacide, pour la préparation d'un médicament, ou vaccin, destiné à la prévention ou au traitement du lupus érythémateux disséminé.

Les peptides hybrides susmentionnés de l'invention, peuvent également être définis par la formule générale suivante (A) :

AA1-AA2-..............-AAn-1)-AAn (A)

dans laquelle :
* AA1 à AAn représentent :
   - un aminoacide correspondant à un résidu aminoacyle situé à la même position dans le peptide ou la protéine parent dont les peptides hybrides sont issus,
   - ou un résidu monomère aza-β³ aminoacyle analogue au résidu aminoacyle initialement présent à la même position dans le peptide ou la protéine parent dont les peptides hybrides sont issus, ledit monomère aza-β³ aminoacyle répondant aux formules (A), (B), ou (C) indiquées ci-dessus, suivant qu'il soit respectivement en position N-terminale, C-terminale, ou dans la chaîne desdits peptides hybrides, et dans lesquelles R₁ est identique à la chaîne latérale de l'aminoacide initial du peptide ou de la protéine parent auquel correspondent ledit monomère aza-β³ aminoacyle,
* et n représente un nombre entier de 4 à environ 100.

L'invention a plus particulièrement pour objet l'utilisation susmentionnée de peptides hybrides de formule (I) suivante :

(I) aa₁-N^{α}haaₘ-aaₙ-N^{α}haaₒ-aaₚ

dans laquelle :
- aa₁, aaₙ et aaₚ représentent un résidu aminoacyle, ou un enchaînement de résidus aminoacyles, correspondant aux résidus aminoacyles présents aux mêmes positions dans le peptide ou la protéine parent dont les peptides hybrides sont issus,
- N^{α}haaₘ et N^{α}haaₒ représentent un résidu monomère aza-β³ aminoacyle, ou un enchaînement de résidus monomères aza-β³ aminoacyles analogues aux résidus aminoacyles initialement présents à la même position dans le peptide ou la protéine parent dont les peptides hybrides sont issus, lesdits monomères aza-β³ aminoacyles répondant aux formules (A), (B), ou (C) indiquées ci-dessus, suivant qu'ils soient respectivement en position N-terminale, C-terminale, ou dans la chaîne desdits peptides hybrides, et dans lesquelles R₁ est identique à la chaîne latérale de l'aminoacide initial du peptide ou de la protéine parent auquel correspondent lesdits monomères aza-β³ aminoacyles,
- 1, m, n, o, et p représentent zéro, ou un nombre entier compris entre 1 et 20, sous réserve que l'un au moins de m ou de o soit différent de zéro, et que le nombre minimum de résidus dans lesdits peptides hybrides de formule (I) soit de 4.

L'invention concerne l'utilisation susmentionnée de peptides hybrides issus de l'épitope 88-99 de l'histone H4 à titre de peptide parent, et correspondant à SEQ ID NO : 1, dont l'un au moins des aminoacides initiaux est substitué par un résidu analogue aza-β³ aminoacide, pour la préparation d'un médicament, ou vaccin, destiné à la prévention ou au traitement du lupus érythémateux disséminé.

A ce titre l'invention concerne plus particulièrement l'utilisation susmentionnée de peptides hybrides issus de l'épitope 88-99 défini ci-dessus, et ayant les formules suivantes :
- SEQ ID NO : 2 (ou peptide E):
   ⁸⁸H₂N-Tyr-Ala-Leu-Lys-Arg-Gln-Gly-Arg-Thr-**N^{α}**-**hLeu**-Tyr-Gly-OH⁹⁹
- SEQ ID NO : 3 (ou peptide C):
   ⁸⁸H₂N-Tyr-Ala-**N^{α}**-**hLeu**-Lys-Arg-Gln-Gly-Arg-Thr-Leu-Tyr-Gly-OH⁹⁹
- SEQ ID NO : 4 (ou peptide A):
   ⁸⁸H₂N-Tyr-**N^{α}**-**hAla**-Leu-Lys-Arg-Gln-Gly-Arg-Thr-Leu-Tyr-Gly-OH⁹⁹
- SEQ ID NO : 5 (ou peptide B):
   ⁸⁸H₂N-Tyr-**N^{α}**-**hAla**-**N^{α}**-**hLeu**-Lys-Arg-Gln-Gly-Arg-Thr-Leu-Tyr-Gly-OH⁹⁹
- SEQ ID NO : 6 (ou peptide D):
   ⁸⁸H₂N-Tyr-Ala-Leu-**N^{α}**-**hLys**-Arg-Gln-Gly-Arg-Thr-Leu-Tyr-Gly-OH⁹⁹
- SEQ ID NO : 7 (ou peptide G):
   ⁸⁸H₂N-Tyr-Ala-Leu-Lys-Arg-Gln-Gly-Arg-Thr-**N^{α}-hLeu-N^{α}-hTyr**-Gly-OH⁹⁹
- SEQ ID NO : 8 :
   ⁸⁸H₂N-Tyr-Ala-Leu-Lys-Arg-Gln-**N^{α}**-**hGly**-Arg-Thr-Leu-Tyr-Gly-OH⁹⁹
- SEQ ID NO : 9 :
   ⁸⁸H₂N-Tyr-Ala-Leu-Lys-Arg-Gln-Gly-**N^{α}-hArg**-Thr-Leu-Tyr-Gly-OH⁹⁹
- SEQ ID NO : 10 :
   ⁸⁸H₂N-Tyr-Ala-Leu-Lys-**N^{α}-hArg-**Gln-Gly-Arg-Thr-Leu-Tyr-Gly-OH⁹⁹
- SEQ ID NO : 11 :
   ⁸⁸H₂N-Tyr-Ala-Leu-Lys-Arg-Gln-Gly-Arg-Thr-Leu-**N^{α}-hTyr**-Gly-OH⁹⁹
- SEQ ID NO : 12 (ou peptide F):
   ⁸⁸H₂N-**N^{α}-hTyr**-Ala-Leu-Lys-Arg-Gln-Gly-Arg-Thr-Leu-Tyr-Gly-OH⁹⁹
- SEQ ID NO : 13 (ou peptide H):
   ⁸⁸H₂N-Tyr-Ala-Leu-Lys-Arg-Gln-Gly-Arg-Thr-Leu-Tyr-**N^{α}-hGly**-OH⁹⁹
- SEQ ID NO : 14 (ou peptide I):
   ⁸⁸H₂N-Tyr-Ala-Leu-Lys-Arg-Gln-Gly-Arg-Thr-**N**^{α}-**hLeu-N**^{α}-**hTyr**-**N**^{α}-**hGly**-OH⁹⁹

L'invention concerne plus particulièrement l'utilisation susmentionnée du peptide hybride de formule SEQ ID NO : 2.

L'invention concerne plus particulièrement l'utilisation susmentionnée du peptide hybride de formule SEQ ID NO : 7.

L'invention a également pour objet les peptides hybrides comprenant au moins un aza-β³ aminoacide, ces peptides hybrides étant des analogues de peptides ou protéines parents, lesdits peptides hybrides comprenant au moins un aminoacide initial du peptide ou de la protéine parent, et etant issus de l'epitope de l'histone H4 à titre de peptide parent, correspondant à SEQ ID NO:1.

L' invention concerne plus particulièrement les peptides hybrides tels que définis ci-dessus, et correspondant à la formule générale suivante (A) :

AA1-AA2-..............-AAn-1)-AAn (A)

dans laquelle :
* AA1 à AAn représentent :
   - un aminoacide correspondant à un résidu aminoacyle situé à la même position dans le peptide ou la protéine parent dont les peptides hybrides sont issus,
   - ou un résidu monomère aza-β³ aminoacyle analogue au résidu aminoacyle initialement présent à la même position dans le peptide ou la protéine parent dont les peptides hybrides sont issus, ledit monomère aza-β³ aminoacyle répondant aux formules (A), (B), ou (C) indiquées ci-dessus, suivant qu'il soit respectivement en position N-terminale, C-terminale, ou dans la chaîne desdits peptides hybrides, et dans lesquelles R₁ est identique à la chaîne latérale de l'aminoacide initial du peptide ou de la protéine parent auquel correspondent ledit monomère aza-β³ aminoacyle,
   l'un au moins de AA1 à AAn représentant un aminoacide du peptide parent, à savoir un résidu aminoacyle situé à la même position dans le peptide ou la protéine parent dont les peptides hybrides sont issus,
* et n représente un nombre entier de 4 à environ 100.

A ce titre l'invention concerne plus particulièrement les peptides hybrides définis ci-dessus de formule (I) suivante :

(I) aa₁-N^{α}haaₘ-aaₙ-N^{α}haaₒ-aaₚ

dans laquelle :
- aa₁, aaₙ et aaₚ représentent un résidu aminoacyle, ou un enchaînement de résidus aminoacyles, correspondant aux résidus aminoacyles présents aux mêmes positions dans le peptide ou la protéine parent dont les peptides hybrides sont issus,
- N^{α}haaₘ et N^{α}haaₒ représentent un résidu monomère aza-β³ aminoacyle, ou un enchaînement de résidus monomères aza-β³ aminoacyles, analogues aux résidus aminoacyles initialement présents à la même position dans le peptide ou la protéine parent dont les peptides hybrides sont issus, lesdits monomères aza-β³ aminoacyles répondant aux formules (A), (B), ou (C) mentionnées ci-dessus, suivant qu'ils soient respectivement en position N-terminale, C-terminale, ou dans la chaîne desdits peptides hybrides, et dans lesquelles R₁ est identique à la chaîne latérale de l' aminoacide initial du peptide ou de la protéine parent auquel correspondent lesdits monomères aza-β³ aminoacyles,
- l, m, n, o, et p représentent zéro, ou un nombre entier compris entre 1 et 20, sous réserve que l'un au moins de m ou de o soit différent de zéro, que le nombre minimum de résidus dans lesdits peptides hybrides de formule (I) soit de 4, et l'un au moins de l, n, ou p soit différent de zéro.

L'invention a plus particulièrement pour objet les peptides hybrides susmentionnés de formules suivantes :
- SEQ m NO : 2 (ou peptide E):
   ⁸⁸H₂N- Tyr-Ala-Leu-Lys-Arg-Gln-Gly-Arg- Thr-**N^{α}-hLeu**-Tyr-Gly-OH⁹⁹
- SEQ ID NO : 3 (ou peptide C):
   ⁸⁸H₂N- Tyr-Ala-**N^{α}-hLeu-**Lys-Arg-Gln-Gly-Arg- Thr-Leu- Tyr-Gly-OH⁹⁹
- SEQ ID NO 4 (ou peptide A):
   ⁸⁸H₂N- Tyr-**N^{α}-hAla**-Leu-Lys-Arg-Gln-Gly-Arg-Thr-Leu-Tyr-Gly-OH⁹⁹
- SEQ ID NO : 5 (ou peptide B):
   ⁸⁸H₂N-Tyr-**N^{α}-hAla**-**N^{α}-hLeu-**Lys-Arg-Gln-Gly-Arg-Thr-Leu-Tyr-Gly-OH⁹⁹
- SEQ ID NO : 6 (ou peptide D):
   ⁸⁸H₂N-Tyr-Ala-Leu-**N^{α}-hLys**-Arg-Gln-Gly-Arg-Thr-Leu-Tyr-Gly-OH⁹⁹
- SEQ ID NO : 7 **(ou peptide G):**
   ⁸⁸H₂N-Tyr-Ala-Leu-Lys Arg-Gln-Gly-Arg-Thr-**N^{α}-hLeu-N^{α}-hTyr**-Gly-OH⁹⁹
- SEQ ID NO : 8 :
   ⁸⁸H₂N- Tyr-Ala-Leu-Lys-Arg-Gln-**N^{α}-hGly**-Arg-Thr-Leu-Tyr-Gly-OH⁹⁹
- SEQ ID NO : 9 :
   ⁸⁸H₂N- Tyr-Ala-Leu-Lys-Arg-Gln-Gly-**N^{α}-hArg-**Thr-Leu-Tyr-Gly-OH⁹⁹
- SEQ ID NO : 10 :
   ⁸⁸H₂N-Tyr-Ala-Leu-Lys-**N^{α}-hArg**-Gln-Gly-Arg-Thr-Leu-Tyr-Gly-OH⁹⁹
- SEQ ID NO : 11 :
   ⁸⁸H₂N-Tyr-Ala-Leu-Lys-Arg-Gln-Gly-Arg-Thr-Leu-**N^{α}-hTyr-**Gly-OH⁹⁹
- SEQ ID NO : 12 (ou peptide F):
   ⁸⁸H₂N-**N^{α}-hTyr**-Ala-Leu-Lys-Arg-Gln-Gly-Arg-Thr-Leu-Tyr-Gly-OH⁹⁹
- SEQ ID NO : 13 (ou peptide H):
   ⁸⁸H₂N-Tyr-Ala-Leu-Lys-Arg-Gln-Gly-Arg-Thr-Leu-Tyr-**N^{α}t-hGly**-OH⁹⁹
- SEQ ID NO : 14 (ou peptide I):
   ⁸⁸H₂N-Tyr-Ala-Leu-Lys-Arg-Ghn-Gly-Arg-Thr-**N^{α}-hLeu-N^{α}-hTyr-N^{α}-hGly**-OH⁹⁹
- SEQ ID NO : 16 (ou peptide A') :

L'invention concerne plus particulièrement encore les peptides hybrides tels que définis ci-dessus de formule suivante:
- SEQ ID NO : 2 (ou peptide E):
   ⁸⁸H₂N-Tyr-Ala-Leu-Lys-Arg-Gln-Gly-Arg-Thr-**N^{α}-hLeu-**Tyr-Gly-OH⁹⁹
- SEQ ID NO : 7 (ou peptide G) :
   ⁸⁸H₂N-Tyr-Ala-Leu-Lys-Arg-Gln-Gly-Arg-Thr-**N^{α}-hLeu-N^{α}-hTyr**-Gly-OH⁹⁹

L'invention concerne également les complexes entre un peptide hybride tel que défini ci-dessus, et un élément du complexe d'histocompatibilité majeur (encore désigné complexe MHC-hybride), et éventuellement un récepteur de cellules T (encore désigné complexe MHC-hybride-récepteur T).

L'invention concerne plus particulièrement un complexe entre un peptide hybride tel que défini ci-dessus, et un récepteur de cellules T.

L'invention a également pour objet une méthode de diagnostic *in vitro* de pathologies associées à la présence dans l'organisme d'un patient, d'une protéine exogène ou endogène, susceptible d'être directement ou indirectement impliquée dans le processus d'apparition et/ou de développement de ces pathologies, caractérisée en ce qu'elle comprend:
- la mise en contact d'un échantillon biologique provenant d'un patient susceptible d'être porteur d'anticorps dirigé contre ladite protéine, avec un peptide hybride tel que défini ci-dessus, ledit peptide hybride étant issu de tout ou partie de ladite protéine endogène ou exogène, ou issu d'un peptide susceptible d'être reconnu par des anticorps reconnaissant eux-mêmes la protéine exogène ou endogène, dans des conditions permettant la réaction entre les anticorps dirigés contre la protéine et susceptibles d'être présents dans l'échantillon biologique, et le susdit peptide hybride;
- la détection *in vitro* du complexe antigène / anticorps susceptible d'être formé à l'étape précédente ou
- la détection *in vitro* d'anticorps circulants chez le patient par un test de compétition en utilisant un anticorps anti-hybride.

L'invention a également pour objet un nécessaire ou kit pour la mise en oeuvre d'une méthode de diagnostic *in vitro* telle que définie ci-dessus, comprenant:
- un peptide hybride issu de tout ou partie de la protéine endogène ou exogène, ou correspondant à un peptide susceptible d'être reconnu par des anticorps reconnaissant eux-mêmes la protéine exogène ou endogène,
- des réactifs pour rendre un milieu apte à la formation d'une réaction immunologique,
- des réactifs permettant de détecter le complexe antigène / anticorps qui a été produit à l'issue de la réaction immunologique, lesdits réactifs contenant éventuellement un marqueur ou étant susceptibles d'être reconnus à leur tour par un réactif marqué, plus particulièrement dans le cas où le peptide hybride ou les anticorps anti-hybrides susmentionnés ne sont pas marqués.

L'invention concerne également les compositions pharmaceutiques, notamment les vaccins, comprenant au moins un peptide hybride tel que défini ci-dessus, en association ou non avec un véhicule physiologiquement acceptable.

L'invention a plus particulièrement pour objet les compositions pharmaceutiques susmentionnées comprenant au moins un peptide hybride tel que défini ci-dessus, associé ou non à une molécule porteuse, protéique ou non, pouvant induire *in vivo* la production d'anticorps neutralisant la protéine exogène ou endogène responsable de la pathologie, ou induire *in vivo* une réponse immune cellulaire cytotoxique ou auxiliaire.

L'invention concerne également les anticorps anti-peptides hybrides polyclonaux ou monoclonaux tels qu'obtenus par immunisation d'un animal avec au moins un peptide hybride défini ci-dessus, lesdits anticorps étant susceptibles de former un complexe avec ces peptides hybrides, et/ou avec les peptides ou protéines parents correspondant à ces derniers, et caractérisés en ce qu'ils reconnaissent le peptide parent ou la protéine parente avec une affinité au moins égale à celle présentée par les anticorps anti-peptide parent ou anti-protéine parente vis à vis du peptide parent ou de la protéine parente.

L'invention a plus particulièrement pour objet les anticorps anti-idiotypes susceptibles de former un complexe avec les anticorps susmentionnés, tels qu'obtenus par immunisation d'un animal avec lesdits anticorps.

L'invention concerne également une méthode de diagnostic *in vitro* de pathologies associées à la présence dans l'organisme d'un patient d'une protéine exogène ou endogène, susceptible d'être directement ou indirectement impliquée dans le processus d'apparition et/ou de développement de ces pathologies, ladite méthode étant caractérisée en ce qu'elle comprend:
- la mise en contact d'un échantillon biologique provenant d'un patient susceptible d'être porteur de ladite protéine, avec l'un au moins des anticorps susmentionnés, les anticorps étant avantageusement dirigés contre un peptide hybride issu de tout ou partie de ladite protéine endogène ou exogène, ou
   dans des conditions permettant la réaction entre la protéine susceptible d'être présente dans l'échantillon biologique, et les susdits anticorps dirigés contre le susdit peptide hybride;
- la détection *in vitro* du complexe antigène / anticorps susceptible d'être formé à l'étape précédente.

L'invention a également pour objet un nécessaire ou kit pour la mise en oeuvre d'une méthode de diagnostic *in vitro* telle que définie ci-dessus, comprenant:
- des anticorps susmentionnés, dirigés contre ce peptide hybride;
- des réactifs pour rendre un milieu apte à la formation d'une réaction immunologique;
- des réactifs permettant de détecter le complexe antigène / anticorps qui a été produit à l'issue de.la réaction immunologique, lesdits réactifs contenant éventuellement un marqueur ou étant susceptibles d'être reconnus à leur tour par un réactif marqué, plus particulièrement dans le cas où le peptide hybride ou les anticorps anti-hybrides susmentionnés ne sont pas marqués.

L'invention concerne également les compositions pharmaceutiques, notamment les vaccins, comprenant au moins un anti-idiotype susmentionné, en association avec un véhicule physiologiquement acceptable.

L'invention a plus particulièrement pour objet les compositions pharmaceutiques susmentionnées comprenant au moins un anti-idiotype tel que défini ci-dessus, associé à une molécule porteuse, protéique ou non, pouvant induire *in vivo* la production d'anticorps neutralisant la protéine exogène ou endogène responsable de la pathologie, ou induire *in vivo* une réponse immune cellulaire cytotoxique.

L'invention concerne également les compositions pharmaceutiques, comprenant des anticorps tels que définis ci-dessus, en association ou non avec un véhicule physiologiquement acceptable.

L'invention sera davantage illustrée à l'aide de la description détaillée qui suit de la synthèse d'aza-β³ aminoacides, et de peptides hybrides les contenant, ainsi que de leur activité biologique.

### Peptides hybrides de l'histone H4

La séquence sur laquelle les Inventeurs ont travaillé dans un premier temps est un peptide de l'histone H4 (résidus 88-99: YALKRQGRTLYG) qui représente un épitope T CD4⁺ immunodominant minimum reconnu par des cellules Th ganglionnaires de souris immunisées contre du nucléosome, structure de base de la chromatine, formée d'ADN et des quatre histones H2A, H2B, H3 et H4. Il a été démontré ces dernières années que le nucléosome joue un rôle primordial en tant qu'antigène et immunogène dans une maladie autoimmune systémique, le lupus érythémateux disséminé, qui touche aujourd'hui 1 million d'Américains. Ce peptide de la région C-terminale de l'histone H4 a l'importante propriété de ne pas être reconnu par des cellules Th générées contre la protéine H4 isolée, mais seulement par les cellules Th générées contre le nucléosome. Des études détaillées de ce peptide chez la souris normale BALB/c et à l'aide d'un modèle murin de lupus (souris NZBxNZW) ont permis d'obtenir des informations concernant la réponse cellulaire T CD4⁺ et B (production d'anticorps) dirigées contre ce peptide.

La synthèse de plusieurs analogues de ce peptide 88-99 de l'histone H4 a été réalisée en remplaçant avec succès différentes positions par leur analogue respectif N^{α}haa, selon la méthodologie décrite ci-dessous.

### A) Méthodologie de Synthèse

La synthèse décrite ci-après est celle d'aza-β³ peptides ou peptides hybrides incluant un ou plusieurs monomères aza-β³ amino acides, homologues azotés d'amino acides. Les chaînes latérales des monomères mimant les aminoacides sont portées par des atomes d'azote qui sont isoélectroniques des CHα, (atomes d'azote chiraux à configuration non fixée), ce qui leur confére une grande liberté conformationnelle. De plus, ces monomères ne possèdent aucun centre d'asymétrie de configuration fixée. Le positionnement correct de la chaîne peptidique dans un site enzymatique se produit à la fois par le déplacement d'équilibres conformationnel et configurationnel. L'action d'un tel composé, d'un point de vue stéréochimique, est équivalente à celle d'un mélange de diastéréoisomères en équilibre rapide, l'interaction avec le site enzymatique déplaçant l'équilibre vers le plus affine.

La méthode de la présente invention permet d'introduire une grande variété de chaînes latérales, protéogéniques ou non protéogéniques, dans des positions choisies. D'autres bénéfices potentiels en résultent également tels qu'une simplification des méthodes de synthèse (suppression des problèmes stéréochimiques) et une plus grande résistance de tels analogues aux squelettes modifiés vis-à-vis de l'action des peptidases. Ceci permet d'une part de mimer la plupart des aminoacides naturels et non naturels et d'autre part d'introduire sur le squelette pseudopeptidique des chaînes latérales susceptibles de moduler ses caractéristiques biophysiques. L'introduction de groupements favorisant le passage de ces analogues à travers les membranes cellulaires (chaînes lipophiles) ou augmentant leur solubilité dans le milieu plasmatique (groupements perfluorés par exemple) nous permet de moduler, voire, d'optimiser la biodisponibilité de ces composés.

### a) Monomères aza-β³-amino acides

Deux méthodologies de synthèse sont utilisées pour obtenir les monomères aza-β³-amino acides à partir des hydrazines convenablement substituées et protégées, selon la nature des chaînes latérales que l'on désire mimer.
- Soit par bromoacétylation d'hydrazines N,N'-disubstituées, la déprotection du monomère orthogonalement protégé conduisant à l'aza β³ amino acide désiré avec des rendements de l'ordre de 60-80 %. 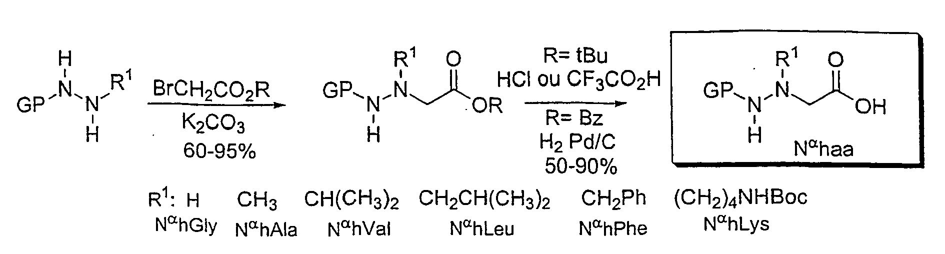
   Cette méthode consistant à réaliser une substitution nucléophile puis une déprotection a été publiée dans Synlett : New Monomers for Solid Phase Synthesis of Hydrazinopeptoides: the Nα-Substituted-Nβ-Protected hydrazinoglycines and Nα-Substituted-Nβ-Protected hydrazinoglycinals. A. Cheguillaume, I. Doubli-Bounoua, M. Baudy-Floc'h, P. Le Grel, Synlett 2000, 3, 331-334.

### - Soit par amination réductrice de l'acide glyoxylique.

Cette méthode est une nouvelle méthode de synthèse des Fmoc-aza-β³amino acides (N^{α}haa): A une suspension d'hydrazine substituée Fmoc protégée (1eq) dans 50ml d'EtOH, de l'acide glyoxylique (1.1eq) est ajouté sous agitation. Après 0.5h, NaBH₃CN (1.2eq) est ajouté au mélange, le pH est ajusté à 3-4 par addition d'HCl 2N, après 0.5h supplémentaire le pH est ajusté à 1. Après 10 min d'agitation le mélange réactionnel est concentré par évaporation, puis dilué par addition de 100 mL d'acétate d'éthyle. La solution est lavée successivement par NaHCO₃ (5%) et de la brine puis séchée sur Na₂SO₄. Après évaporation du solvant, on obtient le monomère Fmoc-aza-β³ amino acide avec des rendements de l'ordre de 82-87%.

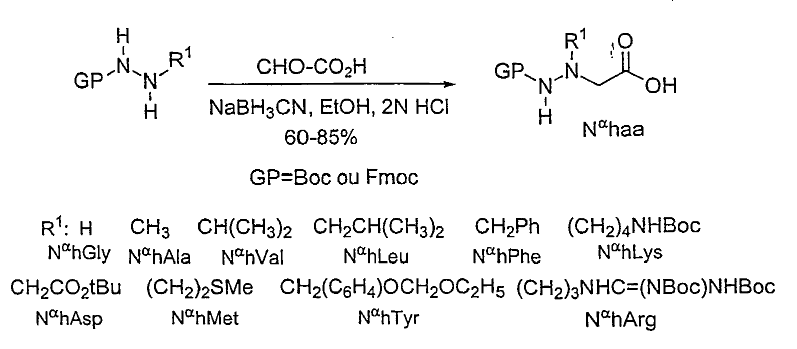

### a-1) Fmoc aza-β³-Glycine (Fmoc-N^{α}hGly-OH)

A une suspension de Fmoc carbazate (1eq) dans 50ml d'EtOH, de l'acide glyoxylique (1.1eq) est ajouté sous agitation. Après 0.5h, NaBH₃CN (1.2 eq) est ajouté au mélange, le pH est ajusté à 3-4 par addition d'HCl 2N, après 0.5h supplémentaire le pH est ajusté à 1. Après 10 min d'agitation le mélange réactionnel est concentré par évaporation, puis dilué par addition de 100 mL d'acétate d'éthyle. La solution est lavée successivement par NaHCO₃ (5%) et de la brine puis séchée sur Na₂SO₄. Après évaporation du solvant, on obtient le monomère Fmoc-aza-β³ glycine avec un rendement de 86%.

mp: 122-124°C. ¹H NMR (DMSO) : 3.75 (s, 2H, CH₂), 4.25 (t, 1H, *J* = 6.8 Hz, CH), 4.50 (d, 2H, J= 6.8 Hz, CH₂), 6.90 (br s, 1H, NH), 7.30-7.85 (m, 8H, Ar), 10.0 (s, 1H, OH). ¹³C NMR (DMSO): 162.90, 144.07, 141.59, 128.06, 127.38, 125.44, 120.30, 67.37, 58.00, 47.43. HRMS: (M+) Calc. pour C₁₇H₁₅N₂O₃ 295.1082; Tr: 295.1083.

### a-2) Fmoc -aza-β³-Aspartique acide (Fmoc-N^{α}hAsp(OtBu)-OH).

**a-2-1) Fmoc NH-NHCH₂CO₂tBu:** A une solution de 2.54g de Fmoc carbazate (10 mmol) dans 10ml DMF, une solution de 1.95g de bromoacetate de tert-butyl (10mmol) dans 10ml de CH₂Cl₂ est ajoutée goutte à goutte sous agitation à température ambiante. Après 12h d'agitation, le solvant est partiellement évaporé et le résidu est purifié par chromatographie sur gel de silice (ethyl acetate / hexane 1/1) pour obtenir 2.79g (rendement: 76%) sous forme d'huile incolore qui cristallise lentement.

mp.114-116°C. ¹H NMR (CDCl₃) 1.50 (s, 9H, tBu), 3.61 (d, 2H, CH₂), 4.25 (t, 1H, J= 6.8 Hz, CH), 4.44 (d, 2H, J= 6.8 Hz, CH₂), 6.90 (brs, 1H, NH), 7.20-7.80 (m, 13H, ar). ¹³C NMR (CDCl₃): 171.55, 162.90, 144.07, 141.59, 128.06, 127.38, 125.44, 120.30, 82.21, 67.37, 53.10, 47.43, 28.42. C₂₁H₂₄N₂O₄ 368.1736 Calc: C, 68.45; H, 6.57; N, 7.61; Tr., C, 68.56; H, 6.73; N, 7.62.

### a-2-2) Fmoc-N^{α}hAsp(OtBu)-OH :

Le monomère **Fmoc-N^{α}hAsp(OtBu)** est préparé en suivant le mode opératoire général d'amination réductrice à partir de l'hydrazine Fmoc NH-NHCH₂CO₂tBu décrit plus haut

87% ; mp: 98-100°C. ¹H NMR (CDCl₃): 1.50 (s, 9H, ^{t}Bu), 3.60 (m, 2H, CH₂), 3.72 (m, 2H, CH₂), 4.22 (t, 1H, J = 6.3 Hz, CH), 4.50 (d, 2H, J = 6.3 Hz, CH₂), 7.20 (brs, 1H, NH), 7.22-7.84 (m, 8H, ar). ¹³C NMR (CDCl₃): 171.23, 171.00, 158.03, 143.61, 141.76, 128.33, 127.59, 125.33, 120.50, 83.80, 68.19, 59.20, 58.80, 47.49, 28.53. HRMS: (M+) Calc.pour C₂₃H₂₆N₂O₆ 426.1790; Tr: 426.1790 Anal. calc: C, 64.76; H, 6.15; N, 6.57; Tr, C, 64.84; H, 6.18; N, 6.58.

### a-3) Fmoc aza-β³-Methionine (Fmoc-N^{α}hMet-OH)

**a-3-1) Fmoc NH-NH(CH₂)₂SMe :** A une solution de Methyl Thio Acetaldehyde Dimethylacetal (5g, 36mmol) dans CH₂Cl₂, 16ml d'HCl (1%) est additionné. Après agitation à température ambiante pendant 0.5h, une suspension de Fmoc cabazate (8.38g, 33mmol) dans 100ml de THF est additionnée. Après 10min, 1g de tamis moléculaire (4Å) est ajouté et le mélange est laissé sous agitation pendant 12h. 2.14g de cyanoborohydrure de sodium (34mmol) sont ensuite ajoutés par fractions sur une période de 45min, le pH étant maintenu à 3-4 par addition par une solution d'HCl 2N. Le mélange est agité pendant 2h supplémentaire, puis ajusté à pH 1. Le mélange est dilué dans 50ml d'acétate d'éthyle, neutralisé par NaHCO₃ et lavé par de la brine. Les phases aqueuses sont extraites par 3x50ml de CH₂Cl₂. Les phases organiques sont rassemblées et séchées sur Na₂SO₄. Le solvant est évaporé et l'huile obtenue est reprise à l'ether de pétrole pour donner un précipité de 4.54g (42%).

mp:132°C. ¹H NMR (CDCl₃): 2.15 (s, 3H, CH₃), 2.64 (t, 2H, J= Hz, CH₂), 3.12 (t, 2H, J = Hz, CH₂), 4.25 (t, 1H, J = 6.6 Hz, CH), 4.52 (d, 2H, J = 6.6 Hz, CH₂), 6.46 (brs, 1H, NH), 7.25-7.82 (m, 8H, ar), 8.21 (brs, 1H, NH). ¹³C NMR (CDCl₃): 157.67, 144.05, 141.75, 128.21, 127.51, 125.39, 120.46, 67.41, 50.09, 47.58, 32.63, 15.66. Anal calc. pour C₁₈H₂₀O₂N₂S 328.1245: C, 65.83; H, 6.14; N, 8.54; S, 9.74. Tr: C, 65.90; H, 6.18; N, 8.62; S, 9.69.

### a-3-2) Fmoc-N^{α}hMet-OH:

Le monomère **Fmoc-N^{α}hMet-OH** est préparé en suivant le mode opératoire général d'amination réductrice à partir de l'hydrazine Fmoc NH-NH(CH₂)₂SMe décrit plus haut

83%. ¹H NMR (CDCl₃): 2.15 (s, 3H, CH₃), 2.55 (t, 2H, J= Hz, CH₂), 3.15 (t, 2H, J = Hz, CH₂), 3.69 (s, 2H, CH₂), 4.20 (t, 1H, *J =* 6.6 Hz, CH), 4.50 (d, 2H, J = 6.6 Hz, CH₂), 6.95 (brs, 1H, NH), 7.25-7.82 (m, 8H, ar), 9.24 (sl, 1H, OH). ¹³C NMR (CDCl₃): 173.42, 156.02, 143.87, 141.76, 128.26, 127.54, 125.38, 120.45, 67.54, 59.18, 56.70, 47.56, 30.12, 16.09. HRMS [M+H]⁺ C₂₀H₂₃N₂O₄S Calc: 387.1379; Tr: 387.1379.

### a-4) Fmoc aza-β³-Tyrosine (Fmoc-N^{α}hTyr(OCH₂OEt)-OH)

**a-4-1) 4-(ethyloxymethyloxy)benzaldehyde**: A un mélange de 4-hydroxybenzaldehyde (5g, 0.041 mol) et de 12ml de triethylamine dans 30ml de THF est ajouté une solution de chloromethyl ethyl ether (5.65g, 0.06mol) dans 20ml THF à 0°C et le mélange esst agité à température ambiante pendant 2h. Le chlorhydrate de triéthylamine est filtré et le solvant est évaporé sous pression réduite pour donner le 4-ethyloxymethyloxy benzaldehyde sous forme d'huile (6.63g, 90%).

¹H NMR (CDCl₃): 1.30 (t, 3H, J = 7.1 Hz, CH₃), 3.70 (q, 2H, J = 7.1 Hz, CH₂), 5.25 (s, 2H, CH₂), 7.15-7.80 (m, 4H, ar), 10.10 (s, 1H, CHO).

**a-4-2) Fmoc-NH-NHCH₂(C₆H₄)OCH₂OEt :** 5.6g de Fmoc cabazate (22mmol) est ajouté sous agitation à une solution de 4-(ethyloxymethyloxy)benzaldehyde (5.3g, 29mmol) dans 100ml de THF sec à température ambiante. Après 10min, 1g de tamis moléculaire (4Å) est ajouté et le mélange est agité pendant 1h. 1.57g de cyanoborohydrure de sodium (25mmol) est ajouté en 45min et le pH est maintenu à 3-4 par addition d'une solution d'HCl (2N). Après 2h d'agitation, le pH est ajusté à 1. 50ml d'acétate d'éthyle sont alors additionné et le mélange est neutralisé par une solution saturée de NaHCO₃. La phase aqueuse est extraite par CH₂Cl₂ (3x50ml). Les phases organiques sont séchées sur Na₂SO₄ et le solvant est évaporé sous pression réduite pour donner une huile qui par addition d'éther de pétrole donne un précipité blanc (5.34g, 58%).

Mp: 113°C. ¹H NMR (CDCl₃): 1.30 (t, 3H, J = 7.1 Hz, CH₃), 3.70 (q, 2H, J = 7.1 Hz, CH₂), 3.98 (d, 2H, J= 6.8Hz, CH₂), 4.25 (t, 1H, J= 6.8 Hz, CH), 4.50 (d, 2H, J= 6.8 Hz, CH₂), 5.25 (s, 2H, CH₂), 6.30 (brs, 1H, NH), 7.15-7.80 (m, 13H, ar), 8.21 (brs, 1H, NH). ¹³C NMR (CDCl₃): 157.49, 144.06, 141.76, 130.70, 128.19, 127.50, 125.40, 120.44, 116.68, 93.58, 67.36, 64.65, 55.48, 47.60, 15.53. Anal.Calc : C₂₅H₂₆N₂O₄: 418.1892. C, 71.74 ; H, 6.27; N, 6.70; Tr : C, 71.78; H, 6.29; N, 6.70.

### a-4-3) Fmoc-N^{α}hTyr (OCH₂OEt)-OH :

Le monomère **Fmoc-N^{α}hTyr (OCH₂OEt)-OH** est préparé en suivant le mode opératoire général d'amination réductrice à partir de l'hydrazine Fmoc-NH-NHCH₂(C₆H₄)OCH₂OEt décrit plus haut

¹H NMR (CDCl₃): 1.25 (t, 3H, *J=* 7.0 Hz, CH₃), 3.68 (q, 2H, *J=* 7.0 Hz, CH₂), 3.70 (s, 2H, CH₂), 4.05 (s, 2H, CH₂), 4.20 (t, 1H, J= 6.8 Hz, CH), 4.50 (d, 2H, J= 6.8 Hz, CH₂), 5.26 (s, 2H, CH₂), 6.80 (brs, 1H, NH), 7.25-7.80 (m, 8H, ar), 8.60 (brs, 1H, NH), 9.80 (br s, 1H, OH). ¹³C NMR (CDCl₃): 173.40, 157.68, 156.02, 143.93, 141.74, 131.04, 128.23, 127.55, 125.43, 120.43, 116.73, 93.48, 66.30, 64.68, 61.11, 59.00, 47.51, 15.61. HRMS [M+H]+ C₂₇H₂₉N₂O₆ Calc: 477.2026; Tr: 477.2023. Anal. Calc: C, 68.04; H, 5.93; N, 5.88; Tr: C, 68.00; H, 5.90; N, 5.87.

### a-5) Fmoc aza-β³ Arginine (Fmoc-N^{α}hArg (Boc)-OH)

**a-5-1) 1-*tert*-Butoxycarbonylamino-3,3-diethoxypropane:** Un mélange de di-tert-butyl dicarbonate (9g, 40 mmol.) dans le dioxane (40ml) est additionné goutte à goutte, à une solution, sous agitation et à 0°C, de 1-amino-3,3-diéthoxypropane (5.52g, 37 mmol) et de Et₃N (4.04g, 40 mmol) dans 5ml dioxane. Après 2h, le mélange est agité à température ambiante 12h puis le solvent est évaporé. L'huile résiduelle est reprise par 10mL d'eau, acidifiée avec 30ml HCl (1%), puis extraite à l'acétate d'éthyle (60ml x 3). Les phases organiques sont séchées (MgSO4) et évaporées pour donner 8.89 g de 1-tert-Butoxycarbonyl-amino-3,3-diethoxypropane (90%).

¹H NMR (CDCl₃): 1.20 (t, 6H, J= 8.8 Hz, CH₃), 1.40 (s, 9H, C(CH₃)₃), 1.60-2.00 (m, 2H, CH₂C), 3.00-3.80 (m, 6H, OCH₂+NHCH₂), 4.50 (t, 1H, J= 6.4 Hz, CH), 5.05-5.10 (br, 1H, NH).

**a-5-2) 3-tert-Butoxycarbonylaminopropanal:** Une solution de 1-tert-Butoxycarbonyl-amino-3,3-diethoxypropane (8.89g, 36mmol) dans 15ml d'acide acétique et 4ml d'eau est agitée à température ambiante pendant 10h, puis neutralisée par NaHCO₃, reprise à l'acétate d'éthyle et lavée à brine. Les phases organiques sont évaporées à pression réduite pour donner 5.17g de 3-tert-butoxycarbonylaminopropanal (83%).

¹H NMR (CDCl₃). 1.45 (s, 9H, C(CH₃)₃), 2.60-2.80 (t, 2H, CCH₂), 3.20-3.50 (m, 2H, NCH₂), 5.10-5.20 (br s, 1H, NH), 9.86 (s, 1H, CHO).

**a-5-3) Fmoc-NH-NH(CH₂)₃ NHBoc:** 5.08g de Fmoc carbazate (20mmol) est additionné à une solution sous agitation de 3-tert-Butoxycarbonylaminopropanal (3.46g, 20mmol) dans 100ml de THF sec à température ambiante. Après 10min, 1g de tamis moléculaire (4Å) est ajouté et le mélange réactionnel est agité pendant 12h. 1.26g de cyanoborohydrure de sodium (20mmol) est ajouté par fractions en 45min. Le pH est maintenu à 3-4 par addition d'une solution d'HCl 2N. Le mélange est agité pendant 2h supplémentaire, puis le pH est ajusté à 1. 50 mL d'acétate d'éthyle sont alors ajouté au mélange, la solution est neutralisée par NaHCO₃. Le mélange est extrait par CH₂Cl₂ (3x50ml). Les phases organiques sont séchées (Na₂SO₄) et le solvant est évaporé pour donner une huile qui cristallise par addition d'éther de pétrole (4.27g, 52%).

mp: 101°C. ¹H NMR (DMSO) 1.40 (s, 9H, tBu), 1.50 (m, 2H, CH₂), 2.68 (m, 2H, CH₂), 2.98 (m, 2H, CH₂), 4.24 (t, 1H, J = 6.9 Hz, CH), 4.32 (d, 2H, J = 6.9 Hz, CH₂), 4.70-4.85 (brs, 1H, NH), 6.78 (brs, 1H, NH), 6.80 (brs, 1H, NH), 7.25-7.90 (m, 8H, ar). ¹³C NMR (DMSO) 157.21, 155.95, 144.18, 142.94, 127.98, 127.64, 125.59, 120.46, 77.71, 65.82, 47.08, 38.39, 28.62, 28.21. HRMS calc C₂₃H₂₉N₃O₄: 411.2158 Anal Calc C₂₃H₂₉N₃O₄: C, 67.12; H, 7.11; N, 10.22. Tr: C, 67.22; H, 7.19; N, 10.24.

### a-5-4) Aza-β³-(Boc)homolysinate de benzyle

A une solution d'hydrazine Fmoc protégée Fmoc-NH-NH(CH₂)₃ NHBoc obtenue lors de l'étape précédente (3.7g, 9mmol) et de 2-bromoacetate de benzyle (2.66g, 11.6mmol) dans 20ml de toluène est ajoutée K₂CO₃ (802mg, 5.8mmol). Le mélange est porté à reflux sous agitation pendant 28h. La solution est filtrée et évaporée sous vide. Le brut est chromatographié sur gel de silice (ethyl acetate/hexane 1/3) pour donner 3.02g (60%) d'aza-β³-(Boc)homolysinate de benzyle sous forme d'huile qui précipite lentement.

mp: 107°C.¹H NMR (CDCl₃) 1.50 (s, 9H, tBu), 1.62 (m, 2H, CH₂), 2.97 (m, 2H, CH₂), 3.24 (m, 2H, CH₂), 3.75 (m, 2H, CH₂), 4.20 (t, 1H, J= 6.9 Hz, CH), 4.50 (d, 2H, *J* = 6.9 Hz, CH₂), 5.19 (s, 2H, CH₂), 6.88 (brs, 1H, NH), 7.25-7.90 (m, 13H, Ar). ¹³C NMR(CDCl₃): 171.23, 156.55, 155.50, 144.13, 141.77, 135.54, 129.13, 129.04, 128.84, 128.14, 127.47, 125.44, 120.40, 79.38, 67.09, 64.47, 57.63, 54.56, 47.67, 38.83, 28.85, 27.83. HRMS calc. : C₃₂H₃₇N₃O₆: 559.2682. Anal calc. C₃₂H₃₇N₃O₆: C, 68.66 ; H, 6.67; N, 7.51. Tr: C, 68.59; H, 6.86; N, 7.28.

### a-5-5) Aza-β³-homolysinate de benzyle

A une solution de d'aza-β³-(Boc)homolysinate de benzyle (0.56g, 1mmol) dans 4 mL de DCM, 2 mL de TFA sont ajoutés et la solution est agitée pendant 6h à température ambiante. Une évaporation sous pression réduite conduit à l'aza-β³-homolysinate de benzyle (0.41g, 92%) sous forme d'huile.

### a-5-6) Aza-β³-arginate (N-Boc) de benzyle

L'aza-β³-homolysinate de benzyle (0.18g, 0.40mmol) en solution dans 5ml de CH₂Cl₂ est lentement additionné à une solution de (BocNH)₂C=NTf (0.16g, 0.41mmol) [(BocHN)₂C=NTf est préparé selon la méthodologie de Feichtinger, K; Zapf, C; Sings, H.L; Goodman, M. J.Org.Chem. 1998, 63, 3804] et de triéthylamine (0.64ml, 0.46mmol). Après agitation à température ambiante pendant 12h, la solution est lavée par une solution saturée de NaHCO₃ (25ml) et la phase aqueuse est extraite par CH₂Cl₂ (3x50ml). Les phases organiques réunies sont séchées sur Na₂SO₄ et concentrées in vacuo. Le brut, purifié par flash chromatographie (ethyl acetate/hexane 1/2), conduit à l'aza-β³-arginate (N-Boc) de benzyle 0.23g (85%).

mp: 70-72°C. ¹H NMR (CDCl₃) 1.39 (s, 18H, tBu), 1.60 (m, 2H, CH₂), 2.80 (m, 2H, CH₂), 3.32 (m, 2H, CH₂), 3.66 (m, 2H, CH₂), 4.24 (t, 1H, *J* = 7.1 Hz, CH), 4.45 (d, 2H, *J=* 7.1 Hz, CH₂), 5.05 (s, 2H, CH₂), 6.95 (brs, 1H, NH), 7.27-7.82 (m, 13H, Ar), 8.28 (brs, 1H, NH), 11.45(brs, 1H, NH). ¹³C NMR (CDCl₃): 171.45, 170.80, 163.97, 156.57, 153.59, 144.17, 141.73, 135.67, 129.06, 128.91, 128.78, 128.09, 127.45, 125.47, 120.34, 83.35, 79.48, 66.93, 60.74, 58.04, 53.89, 47.64, 38.83, 28.67, 28.44, 27.45. HRMS C₃₈H₄₇N₅O₈ Calcd.701.3424 Anal. Calcd for C₃₈H₄₇N₅O₈ C, 65.02; H, 6.75; N, 9.98. Found: C, 65.10; H, 6.83; N, 9.98.

### a-5-7) FmocN^{α}hArg(Boc)-OH :

30mg de Pd/C à 10% sont ajoutés à une solution d'aza-β³-arginate (N-Boc) de benzyle (0.35g, 0.5mmol) dans 25ml d'éthanol sous atmosphère d'hydrogène. Le mélange est agité à température ambiante pendant 6h. Le catalyseur est filtré sur celite. La célite est lavé par EtOH (3x15ml) et le filtrat est évaporé pour conduire à 0.26g (89%) d'aza-β³-arginine (N-Boc) sous forme d'huile qui cristallise lentement.

Mp: 94°C. ¹H NMR (CDCl₃) 1.38 (s, 9H, tBu), 1.39 (s, 9H, tBu), 1.62 (m, 2H, CH₂), 2.89 (m, 2H, CH₂), 3.38 (m, 2H, CH₂), 3.60 (m, 2H, CH₂), 4.14 (t, 1H, *J* = 7.1 Hz, CH), 4.40 (d, 2H, *J* = 7.1 Hz, CH₂), 7.27-7.82 (m, 8H, Ar), 7.95 (br s, 1H, NH), 8.50 (br s, 1H, NH), 11.50 (br s, 1H, NH). ¹³C NMR (CDCl₃): 171.35, 170.70, 163.90, 157.45, 153.57, 143.96, 141.75, 128.18, 127.52, 127.31, 125.44, 120.38, 83.35, 79.60, 67.42, 58.04, 53.89, 47.61, 38.83, 28.59, 28.46, 27.47. HRMS calc pour C₃₁H₄₁N₅O₈ (M+) 611.2955. Tr (M+) 611.2958. Anal. Calc. for C₃₁H₄₁N₅O₈: C, 60.85; H, 6.76; N, 11.45. Tr. C, 60.95; H, 6.78; N, 11.47.

### a-6) Fmoc aza-β³ -Lysine (Fmoc-N^{α}hLys-OH)

**a-6-1) 1-*tert*-Butoxycarbonylamino-3,3-diethoxybutane:** Un mélange de di-tert-butyl dicarbonate (9.6 g, 40 mmol.) dans le dioxane (40ml) est additionné goutte à goutte, à une solution, sous agitation et à 0°C, de 1-amino-3,3-diéthoxybutane (5.9, 37 mmol) et de Et₃N (4.04g, 40 mmol) dans 5ml dioxane. Après 2h, le mélange est agité à température ambiante 12h puis le solvent est évaporé. L'huile résiduelle est reprise par 10mL d'eau, acidifiée avec 30ml HCl (1%), puis extraite à l'acétate d'éthyle (60ml x 3). Les phases organiques sont séchées (MgSO4) et évaporées pour donner 9.4 g de 1-tert-Butoxycarbonyl-amino-3,3-diethoxybutane (90%).

¹H NMR (CDCl₃): 1.20 (t, 6H, J = 8.8 Hz, CH₃), 1.40 (s, 9H, C(CH₃)₃), 1.60-1.80 (m, 2H, CH₂C), 3.15 (m, 2H NCH₂), 3.48 (m, 2H, OCH₂), 3. 85 (m, 2H, OCH₂), 4.45 (t, 1H, J= 6.4 Hz, CH), 4.65-4.70 (br, 1H, NH).

**a-6-2) 3-tert-Butoxycarbonylaminobutanal :** Une solution de 1-tert-Butoxycarbonyl-amino-3,3'-diethoxybutane (1.61g, 6.2 mmol) dans 12ml d'acide acétique et 6ml d'eau est agitée à température ambiante pendant 5h, puis neutralisée par NaHCO₃, reprise à l'acétate d'éthyle et lavée à brine. Les phases organiques sont évaporées à pression réduite pour donner 1.27g d'une huile correspondant au 3-tert-butoxy carbonylaminobutanal en équilibre avec l'hydroxy-2 pyrazolidine.

¹H NMR (CDCl₃): 1.40 (s, 9H, C(CH₃)₃), 1.70-2.00 (m, 4H, CH₂), 3.20-3.50 (m, 2H, CH₂), 5.30-5.40 (br s, 1H, NH), 9.86 (s, CHO).

**a-6-3) Fmoc-NH-NH(CH₂)₄ NHBoc:** 5.08g de Fmoc carbazate (20mmol) est additionné à une solution sous agitation de 3-tert-Butoxycarbonylaminobutanal (3.46g, 20mmol) dans 100ml de THF sec à température ambiante. Après 10min, 1g de tamis moléculaire (4Å) est ajouté et le mélange réactionnel est agité pendant 12h. 1.26g de cyanoborohydrure de sodium (20mmol) est ajouté par fractions en 45min. Le pH est maintenu à 3-4 par addition d'une solution d'HCl 2N. Le mélange est agité pendant 2h supplémentaire, puis le pH est ajusté à 1. 50 mL d'acétate d'éthyle sont alors ajouté au mélange, la solution est neutralisée par NaHCO₃. Le mélange est extrait par CH₂Cl₂ (3x50ml). Les phases organiques sont séchées (Na₂SO₄) et le solvant est évaporé pour donner une huile qui cristallise par addition d'éther de pétrole (4.27g, 52%).

84%. mp: 149°C ¹H NMR:(DMSO): 1.45 (s, 9H, tBu), 1.55 (m, 4H, 2CH₂), 2.90 (m, 2H, CH₂), 3.20 (m, 2H, CH₂), 4.25 (t, 1H, *J =* 6.8 Hz, CH), 4.50 (d, 2H, *J =* 6.8 Hz, CH₂), 4.65 (br s, 1H, NH), 6.45 (br s, 1H, NH), 7.30-7.85 (m, 8H, ar). ¹³C NMR (DMSO): 158.53, 156.06, 144.13, 141.07, 128.00, 127.41, 125.55, 120.44, 77.73, 65.82, 50.05, 47.05, 40.52, 28.58, 27.47, 24.93. Anal. Calc : C₂₄H₃₁N₃O₄ 425.2314 C, 67.73; H, 7.35; N, 9.88; Tr: C, 67.70; H, 7.33; N, 9.87.

### a-6-4) Fmoc-N^{α}hLys (Boc)-OH:

Le monomère **Fmoc-N^{α}hLys(Boc)-OH** est préparé en suivant le mode opératoire général d'amination réductrice à partir de l'hydrazine **Fmoc**-NH-NH(CH₂)₄ NHBoc décrit plus haut

82%.¹H NMR (CDCl₃): 1.45 (s, 9H, tBu), 1.55 (m, 4H, 2 CH₂), 3.10 (m, 2H, CH₂), 3.60 (m, 2H, CH₂), 4.25 (t, 1H, J= 6.8 Hz, CH), 4.50 (d, 2H, J= 6.8 Hz, CH₂), 4.80 (br s, 1H, NH), 6.90 (br s, 1H, NH), 7.30-7.85 (m, 8H, ar). ¹³C NMR (CDCl₃): 170.97, 156.49, 156.00, 144.17, 141.76, 128.84, 128.14, 125.48, 120.40, 79.38, 67.04, 58.00, 56.52, 47.64, 40.53, 28.83, 27.68, 25.01. HRMS C₂₆H₃₃N₃O₆ [M+Na]⁺ Calc: 506.2267; Tr: 506.2265.

### a-7) Fmoc-aza-β³-Asparagine (Fmoc-N^{α}hAsn(Trt)-OH),

**a-7-1) Bo**c **NH-NHCH₂CONH₂:** A une solution de glyoxalate de méthyle (3 g ; 33,6mmol) dans le DCM (50mL) est ajouté du Boc carbazate (4,23g ; 0,95 eq). Le milieu réactionnel est laissé 12h sous agitation à température ambiante et est concentré pour donner un solide pâteux directement réduit par hydrogénation catalytique dans le méthanol (30mL) en présence de Pd/C (200mg) pendant 20h. Le milieu réactionnel est concentré puis une solution de méthanol ammoniacal 6N (13mL) est ajoutée. Le milieu réactionnel est laissé sous agitation pendant 48h à température ambiante puis est concentré. En présence d'éther diéthylique l'huile précipite pour donner le produit attendu sous forme d'un solide blanc (4,27g, 70%).

RMN 1H (CDCl₃) δ ppm: 1,49 (s, 9H, CH₃) ; 3,56 (s, 2H, CH₂) ; 5,88 (brs, 1H, NH) ; 6,43 (s, 1H, NH amide) ; 7,33 (s, 1H, NH amide).

**a-7-2) Boc-Aza-β³-Asn-OBn :** A une solution d'amide (7,1g ; 37mmol) dans un mélange 1/1 toluène/DMF (70mL) est ajouté successivement le K₂CO₃ (4,6g ; 0,7eq) et le bromoacétate de benzyle (17,10g ; 2eq). Le milieu réactionnel est laissé sous agitation à 50°C pendant 5 jours puis il est concentré et repris avec 100mL de DCM. Après deux lavages à l'eau (2x50mL). La phase organique est séchée sur Na₂SO₄ puis est concentrée pour donner une pâte. La trituration dans l'éther diéthylique permet d'obtenir une poudre blanche (5,35g ; 42%) correspondant au produit attendu.

RMN 1H (CDCl₃) δ ppm: 1,45 (s, 9H, CH₃) ; 3,61 (s, 2H, CH₂) ; 3,77 (s, 2H, CH₂) ; 5,21 (s, 2H, CH₂) ; 5,50 (brs, 1H, NH) ; 6,94 (s, 1H, NH amide) ; 7,33-7,45 (m, 5H, Ar); 8,18 (s, 1H, NH amide).

RMN 13C (CDCl₃) δ ppm: 28,2 (CH₃); 58,58 (CH₂N) ; 60,89 (CH₂N) ; 67,03 (CO₂CH₂) ; 81,23 (C(CH₃) ; 128,47 128,71 128,75 134,92 (C Ar) ; 155,75 (Boc CO) ; 170,32 (CO₂Bn); 172,68 (CONH₂).

**a-7-3) Fmoc-Aza-β³-Asn-OBn :** On fait buller du HCI gazeux dans une solution de BocAza-β³-AsnOBn (6,40g) dans 100mL de DCM. Le milieu réactionnel est laissé sous agitation pendant 1 nuit à température ambiante puis est concentré. Le solide blanc obtenu est trituré dans l'éther puis est filtré. Il est mis en présence de triéthylamine (2,30g ; 1,2eq) dans le DCM (60mL). La solution limpide est concentrée pour donner un solide pâteux directement solubilisé dans un mélange eau/THF 1/1 (100mL). NaHCO₃ (3,19g; 2eq) est ajouté ainsi qu'une solution de FmocCl (5,88g ; 1,2eq) dans le THF (50mL) goutte à goutte. Le milieu réactionnel est laissé sous agitation pendant 24h à température ambiante. Après ajout d'éther diéthylique (75mL) la phase organique est récupérée et est lavée avec une solution aqueuse saturée en NaCl. La phase organique est séchée sur Na₂SO₄ puis est concentrée pour donnée une huile marron. Le produit brut est purifié par chromatographie sur gel de silice (DCM/AcOEt 3/7 et 1/9) pour récupérer le produit attendu sous la forme d'une poudre blanche (g ; %).

RMN ¹H (CDCl₃) δ ppm: 3,62 (s, 2H, CH₂) ; 3,76 (s, 2H, CH₂) ; 4,21 (t, 1H, CH Fmoc) ; 4,47 (d, 2H, CH₂ Fmoc) ; 5,23 (s, 2H, CH₂) ; 5,42 (brs, 1H, NH); 7,16 (s, 1H, NH amide) ; 7,28-7,56 (m, 9H, Ar); 7,56 (d, 2H, Ar) ; 7,78 (d, 2H, Ar) ; 7,94 (s, 1H, NH amide).

RMN ¹³C (CDCl₃) δ ppm: 49,24 (CH Fmoc) ; 60,51 (CH₂N) ; 62,80 (CH₂N) ; 69,19 (CH₂ Fmoc) ; 69,37 (CO₂CH₂); 122,17 127,09 129,24 129,96 130,54 130,63 130,66 130,71 137,00 143,44 145,58 (C Ar) ; 158,56 (Fmoc CO) ; 172,29 (CO₂Bn) ; 174,32 (CONH₂).

**a-7-4) Fmoc-Aza-β³-Asn(NHTrt)-OBn :** A une solution de Fmoc-Aza-β³-Asn(NHTrt)-OBn (180mg, 0,4mmol) dans 2mL d'AcOH sont ajoutés successivement le triphénylinéthanol (102mg, 1eq), l'anhydride acétique (80mg, 2eq) et 2µL d'H₂SO₄ concentré. Le milieu réactionnel, sous agitation, est porté à 55°C pendant 1 heure puis est laissé refroidir à température ambiante. Le milieu réactionnel est concentré à son 1/3 pour ajouté 10mL d'eau glacial. Après extraction à l'AcOEt, la phase organique est lavée à l'eau et avec une solution aqueuse saturée en NaCl. La phase organique est séchée sur Na₂SO₄ puis est concentrée. L'huile obtenue est purifiée par chromatographie sur gel de silice (AcOEt/EP) pour récupérer 140mg (50%) de produit attendu sous la forme d'un solide blanc.

RMN 1H (CDCl₃) δ ppm: 3,70 (s, 2H, CH₂) ; 3,82 (s, 2H, CH₂) ; 4,12 (t, 1H, CH Fmoc) ; 4,26 (d, 2H, CH₂ Fmoc) ; 5,24 (s, 2H, CH₂) ; 7,28-7,60 (m, 26H, Ar); 7,81 (d, 2H, Ar) ; 9,47 (s, 1H, NH).

### a-8) Fmoc -aza-β³-Proline (Fmoc-N^{α}hPro-OH).

**a-8-1) ZNH-NHBoc:** A une solution de Boc carbazate (7.93g, 60mmol) dans 60mL de CHCl₃ est ajoutée une solution aqueuse de NaOH (2.4g, 1eq dans 60mL d'eau). Le mélange est refroidi dans un bain de glace et une solution de benzylchloroformate (10.24g 1eq) dans CHCl₃ (60mL) est additionnée lentement , puis le mélange est maintenu à température ambiante sous agitation pendant 12h. La phase organique est ensuite lavée à l'eau, à la brine puis séchée sur Na₂S0₄. Après évaporation du solvant le solide blanc obtenu est repris à l'éther de pétrole et filtré (14.17g, 89%).

**a-8-2) Benzyl-pyrazolidine-1-carboxylate:** A une solution d'hydrazine bis protégée (5.33g, 20mmol) dans le DMF (40mL), NaH (80% in oil, 1.21g, 2.1eq) est ajouté à 0°C. Après agitation du milieu réactionnel pendant 30 min à temperature ambiante, le 1,3-dibromopropane (4.04g, 1eq) est ajouté et le mélange est agité pendant 12h. Le mélange est verse dans l'eau (80mL) et extrait deux fois avec AcOEt (2x75mL). La phase organique est lavée à l'eau, à la brine et séchée sur Na₂SO₄. Après evaporation du solvant, le mélange est purifié sur colonne par chromatographie sur gel de silice (PE-AcOEt 75-25) et conduit à 5.00g de benzyl tert-butyl pyrazolidine-1,2-dicarboxylate (82%), qui est ensuite déprotégé par dissolution (5.00g, 16.3mmol) dans 50mL DCM et saturation en HClg. Après 12h d'agitation à tempétature ambiante, le milieu est concentré et repris par 30mL d'eau. AcOEt (70mL) est ajouté et la solution est neutralisée par addition d'une solution saturée de NaHCO₃. La phase organique est lavée à l'eau, séchée sur Na₂SO₄, et concentrée pour donner une huile incolore (3.30g, 98%).

**a-8-3) Z-Aza-β³-Proline *tert*-butyl ester:** A une solution de benzyl-pyrazolidine-1-carboxylate (3.30g, 16mmol) dans le toluène (40mL) est ajouté K₂C0₃ (1.55g, 0.7eq) et du bromoacetate *de tert*-butyl (4.68g, 1.5eq). Le mélange est agité pendant 4 jours à 75°C puis filter. Le filtrat est ensuite lave à l'eau, à la brine, puis séché sur Na₂SO₄. Après evaporation du solvant, l'huile obtenue est purifiée sur une colonne de chromatographie de gel de silice (PE-AcOEt 70-30) pour donner 4.35g de Z -Aza-β³-Proline *tert*-butyl ester sous forme d'huile (85%).

¹H NMR (CDCl₃): 1.49 (s, 9H, CH₃), 2.16 (q, 2H, CH₂), 3.24 (t, 2H, CH₂), 3.51 (s, 2H, CH₂), 3.65 (t, 2H, CH2), 5.22 (s, 2H, CH₂), 7.30-7.49 (m, 5H, Ar).

¹³C NMR (CDCl₃) : 26.11 CH₂, 29.74 CH₃, 47.09 54.97 60.22 N-CH₂, 68.85 CH₂, 83.06 C(CH₃)₃, 127.83 127.89 128.40 138.37 Car, 157.05 COCH₂, 170.39 CO.

**a-8-4) Aza-β³-Proline *tert*-butyl ester :** 10% Pd/C (80mg) est ajouté à une solution de Z -Aza-β³-Proline *tert*-butyl ester (1.00g, 3.12mmol) dans MeOH (10mL). Le mélange est laissé sous agitation et sous atmosphère d'hydrogène pendant 12h (évolution contrôlé par TLC). Le catalyseur est filtré sur célite et le filtrat est évaporé , on obtient 0.58g (99%) d'Aza-β³-Proline *tert-*butyl ester sous forme d'huile.

¹H NMR (CDCl₃): 1.50 (s, 9H, CH₃), 2.01 (q, 2H, CH₂), 2.90 (t, 2H, CH₂), 3.03 (t, 2H, CH2), 3.39 (brs, 1H, NH), 3.51 (s, 2H, CH₂).

**a-8-5) Fmoc-Aza-β³-Proline *tert-butyl* ester:** A une solution d'Aza-β³-Proline *tert-*butyl ester (0.58g, 3.12mmol) dans THF/eau (10 / 5 mL), sous agitation est ajouté NaHCO₃ (0.52g, 2eq) puis goutte à goutte, une solution de FmocCl (0.97g, 1.2eq) dans le THF (10mL). Après agitation pendant 12h à température ambiante de l'éther (50mL) est ajouté et la phase organique est prélevée, lavée à la brine, séchée sur Na₂SO₄, puis évaporée. L'huile obtenue est purifiée sur colonne de chromatographie de gel de silice (EP/EtOAc 9/1 and 6/4). On obtient 1.00g (79%) de Fmoc-Aza-β³-Proline *tert*-butyl ester sous forme d'huile.

¹H NMR (CDCl₃): 1.52 (s, 9H, CH₃), 2.18 (q, 2H, CH₂), 3.27 (t, 2H, CH₂), 3.55(s, 2H, CH₂), 3.65 (t, 2H; CH₂), 4.33 (t, 1H, CH Fmoc), 4.46 (d, 2H, CH₂ Fmoc), 7.30-7.88 (m, 8H, Ar).

**a-8-6) Fmoc-Aza-β³-Proline:** Une solution de Fmoc-Aza-β³-Proline *tert*-butyl ester (1.00g, 2.5mmol) dans 10mL de DCM est saturée en HClg puis agitée à température ambiante pendant 5h. Le solvant est évaporé, le mélange est repris par de l'eau (20mL), puis l'on ajoute une solution NaHCO₃ (N) jusqu'à pH basique. La phase aqueuse est extraite à l'éther, acidifiée par HCl 2N, puis extraite à l'AcOEt. La phase organique est lavée à la brine séchée sur Na₂SO₄. Après évaporation du solvant le solide obtenu est trituré dans l'éther de pétrole , on obtient la Fmoc-Aza-β³-Proline (0.64g, 74%) sous la forme d'un solide blanc. Mp : 130°C

¹H NMR (CDCl₃) : 2.01 (q, 2H, CH₂), 2.89 (t, 2H, CH₂), 3.34(s, 2H, CH₂), 3.35 (t, 2H, CH₂), 4.15 (t, 1H, CH Fmoc), 4.47 (d, 2H, CH₂ Fmoc), 7.30-7.88 (m, 8H, Ar).

¹³C NMR (CDCl₃) : 24.10 CH₂, 43.47 N-CH₂, 45.94 CH Fmoc, 54.12 59.98 N-CH₂, 67.08 CH₂ Fmoc, 118.80, 123.63, 125.95, 126.67, 140.13, 142.12 Car, 157.15 CO Fmoc, 169.70 COOH.

HRMS [M+Na]⁺ C₂₀H₂₀N₂O₄Na calc:375.13208; tr. 375.1320.

### b) Synthèse peptidique: Peptides Hybrides

Les monomères décrits ci-dessus peuvent, à l'instar d'un amino acide protégé, être intégrés dans des positions choisies d'un peptide par synthèse sur support solide à l'aide d'un automate de synthèse à stratégie Fmoc afin d'obtenir des peptides hybrides. Ils peuvent également être associés pour conduire à des oligomères constitués exclusivement d'unités aza-β³-amino acides.

La synthèse des peptides hybrides a été effectuée selon le schéma suivant :

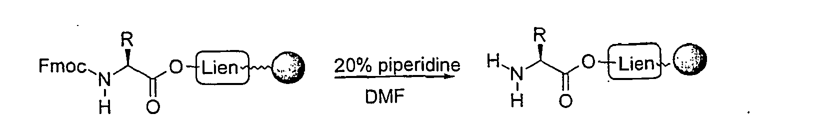

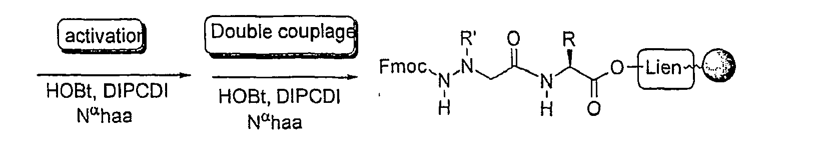

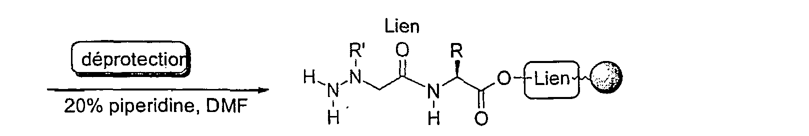

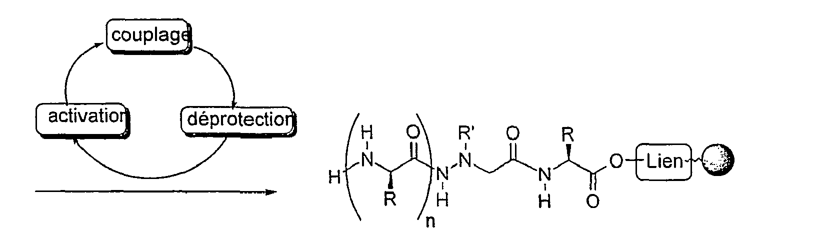

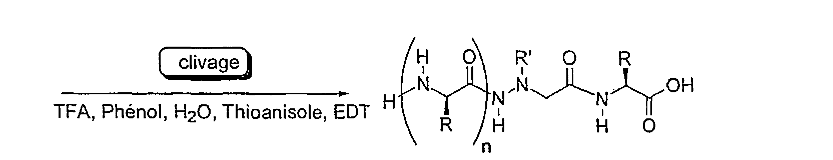

La synthèse des analogues peptidiques a été réalisée à l'aide d'un synthétiseur automatique modèle PepSyntheziser^{™} 9050, Milligen, fonctionnant en stratégie Fmoc en condition de flux continu. Les groupements fonctionnels des chaînes latérales des Fmoc-aminoacides sont protégés par les groupements protecteurs suivants: un groupement t-butoxycarbonyle (Boc) pour la Lysine (Lys), *t*-Butyl (tBu) pour la Tyrosine (Tyr) et la Thréonine (Thr), triphényhnéthyle (Trt) pour la Glutamine (Gln) et 2,2,4,5,6,-pentaméthyldihydrobenzofuran-5-sulfonyle (Pbf) pour l'Arginine (Arg). Le DMF ne doit contenir aucune amine susceptible de déprotéger le groupement Fmoc au cours de la synthèse et la pipéridine utilisée pour les étapes de déprotection est pure à 99%. Le diisopropylcarbodimide (DIPCDI) et le 1-hydroxybenzotriazole (HOBt) sont utilisés en tant qu'agents de couplage.

La résine (1.00 g) préchargée (à - 0.2 mmol/g) par un résidu amino acide ou aza-β³-aminoacide est placée dans le réacteur du synthétiseur. Les couplages sont réalisés avec quatre fois la stoechiométrie en amino acide ou en aza-β³-aminoacide et un temps de couplage de 30 mn, et selon les amino acides ou les aza-β³-aminoacides utilisés, un double couplage ou un temps de couplage de 60 mn est nécessaire. Après chaque étape de couplage et en fin de synthèse, l'extrémité N-terminale du dernier résidu greffé est déprotégée automatiquement par une solution à 20% de pipéridine dans le DMF.

Le clivage de la résine et la déprotection des groupements fonctionnels des chaînes latérales sont réalisés simultanément par action du réactif K (82.5 % TFA, 5 % phénol, 5 % eau, 5 % thioanisole et 2.5 % éthanedithiole). La résine est extraite du réacteur et rincée au dichlorométhane, puis séchée au dessiccateur. Elle est placée dans un ballon puis le cocktail de clivage K fraîchement préparé est ajouté et le milieu réactionnel est laissé sous agitation pendant 3 h à température ambiante. La solution contenant le peptide hybride est ensuite récupérée par filtration de la résine sur fritté. Après évaporation du solvant sous pression réduite jusqu'à un volume d'environ 2 mL, le peptide hybride brut est isolé par précipitation dans l'éther glacé et filtration sur fritté. Il est purifié par HPLC sur une colonne phase inverse C18 (250 x 4.6 mm) selon un gradient d'élution (solvant A : eau + TFA 0.1 % et solvant B acétonitrile + TFA 0.08 %) 0% B à 70% B en 20min puis 70% B à 0% B en 5 min, avec un débit de 1.2 mL / min. La détection UV est réalisée à 210 nm. La pureté des peptides hybrides synthétisés est contrôlée en spectrométrie de masse par la technique ESI dans un mélange acétonitrile / eau (50 / 50).

### 88-99 H4

La synthèse d'analogues du peptide 88-99 de l'histone H4, pour lequel nous avons remplacé avec succès différentes positions, a été réalisée selon cette méthodologie. Pour exemple les monomères Ala, Leu, Lys, Tyr, Gly ont été remplacés par leur analogue respectif N^{α}hAla, N^{α}hLeu, N^{α}hLys, N^{α}hTyr, N^{α}hGly etc ...

Peptide 88-99 de l'histone H4:
H-Tyr-Ala-Leu-Lys-Arg-Gln-Gly-Arg-Thr-Leu-Tyr-Gly-OH

Exemples de peptides hybrides préparés:
- SEQ ID NO : 2 (ou peptide E):
   ⁸⁸H₂N-Tyr-Ala-Leu-Lys-Arg-Gln-Gly-Arg- Thr-**N^{α}-hLeu**-Tyr-Gly-OH⁹⁹
   [M+H]⁺ = 1440.8 ([M+H]⁺ théorique = 1440.8) et pic de l'ion dichargé [M+2H]⁺⁺ = 720.9.
- SEQ ID NO: 3 (ou peptide C):
   ⁸⁸H₂N-Tyr-Ala-**N^{α}-hLeu-**Lys-Arg-Gln-Gly-Arg-Thr-Leu-Tyr-Gly-OH⁹⁹
   [M+H]⁺ = 1440.8 ([M+H]⁺ théorique = 1440.8) et pic de l'ion dichargé [M+2H]⁺⁺ = 720.9.
- SEQ ID NO : 4 (ou peptide A):
   ⁸⁸H₂N-Tyr-**N^{α}**-**hAla**-Leu-Lys-Arg-Gln-Gly-Arg-Thr-Leu-Tyr-Gly-OH⁹⁹
   [M+H]⁺ = 1440.8 ([M+H]⁺ théorique =1440.8) et pic de l'ion dichargé [M+2H]⁺⁺ = 720.9.
- SEQ ID NO : 5 (ou peptide B):
   ⁸⁸H₂N- Tyr-**N^{α}**-**hAla**-**N^{α}**-**hLeu**-Lys-Arg-Gln-Gly-Arg- Thr-Leu-Tyr-Gly-OH⁹⁹
   [M+H]⁺ = 145-5.8 ([M+H]⁺ théorique = 1440.8) et pic de l'ion dichargé [M+2H]⁺⁺ = 728.4.
- SEQ ID NO : 6 (ou peptide D):
   ⁸⁸H₂N- Tyr-Ala-Leu-**N^{α}**-**hLys**-Arg-Gln-Gly-Arg- Thr-Leu- Tyr-Gly-OH⁹⁹
   [M+H]⁺ = 1440.8 ([M+H]⁺ théorique =1440.8) et pic de l'ion dichargé [M+2H]⁺⁺ = 720.9.
- SEQ ID NO : 7 **(ou peptide G)** :
   ⁸⁸H₂N-Tyr-Ala-Leu-Lys-Arg-Gln-Gly-Arg-Thr-**N^{α}-hLeu-Nα-hTyr**-Gly-OH⁹⁹
   [M+H]⁺ = 1455.8 ([M+H]⁺ théorique = 1440.8) et pic de l'ion dichargé [M+2H]⁺⁺ = 728.4.
- SEQ ID NO : 8 :
   ⁸⁸H₂N- Tyr-Ala-Leu-Lys-Arg-Gln-**N^{α}**-**hGly**-Arg-Thr-Leu-Tyr-Gly-OH⁹⁹
   [M+H]⁺ = 1440.8 ([M+H]⁺ théorique = 1440.8) et pic de l'ion dichargé [M+2H]⁺⁺ = 720.9.
- SEQ ID NO : 9 :
   ⁸⁸H₂N-Tyr-Ala-Leu-Lys-Arg-Gln-Gly-**N^{α}-hArg**-Thr-Leu-Tyr-Gly-OH⁹⁹
   [M+H]⁺ = 1440.8 ([M+H]⁺ théorique = 1440.8) et pic de l'ion dichargé [M+2H]⁺⁺ = 720.9.
- SEQ ID NO : 10 :
   ⁸⁸H₂N- Tyr-Ala-Leu-Lys-**N**^{α}-**hArg**-Gln-Gly-Arg- Thr-Leu- Tyr-Gly-OH⁹⁹
   [M+H]⁺ = 1440.8 ([M+H]⁺ théorique = 1440.8) et pic de l'ion dichargé [M+2H]⁺⁺ = 720.9.
- SEQ ID NO : 11 :
   ⁸⁸H₂N- Tyr-Ala-Leu-Lys-Arg-Gln-Gly-Arg- Thr-Leu-**N^{α}**-**hTyr**-Gly-OH⁹⁹
   [M+H]⁺ = 1440.8 ([M+H]⁺ théorique =1440.8) et pic de l'ion dichargé [M+2H]⁺⁺ =720.
- SEQ ID NO : 12 (ou peptide F):
   "H₂N-**N^{α}-hTyr**-Ala-Leu-Lys-Arg-Gln-Gly-Arg-Thr-Leu-Tyr-Gly-OH⁹⁹
   [M+H]⁺ = 1440.8 ([M+H]⁺ théorique = 1440.8) et pic de l'ion dichargé [M+2H]⁺⁺ = 720.9.
- SEQ ID NO : 13 (ou peptide H):
   ⁸⁸H₂N-Tyr-Ala-Leu-Lys-Arg-Gln-Gly-Arg- Thr-Leu- Tyr-**N**^{α}-**hGly**-OH⁹⁹
   [M+H]⁺ = 1440.8 ([M+H]⁺ théorique = 1440.8) et pic de l'ion dichargé [M+2H]⁺⁺ = 720.9.
- SEQ ID NO : 14 (ou peptide I):
   ⁸⁸H₂N-Tyr-Ala-Leu-Lys-Arg-Gln-Gly-Arg-Thr-**N^{α}-hLeu-N^{α}-hTyr-N^{α}-hGly**-OH⁹⁹
   [M+H]⁺ = 1470.8 ([M+H]⁺ théorique = 1470.8) et pic de l'ion dichargé [M+2H]⁺⁺= 735.9.

### B) Analyses biologiques

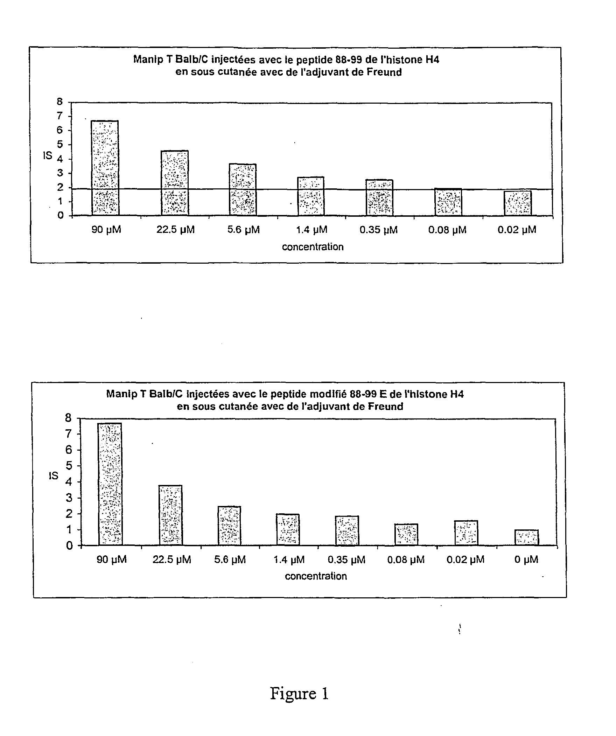

Les souris BALB/c ont été immunisées avec le peptide parent 88-99 H4 et dans les cultures les lymphocytes T de ces souris ont été restimulés avec le même peptide ou avec les analogues A-E. La prolifération cellulaire a été mesurée par l'incorporation de thymidine tritiée et l'indice de stimulation (IS) par rapport aux puits sans peptide a été calculé. Les tests sont réalisés en triplicat et l'expérience est réalisée plusieurs fois dans des expériences indépendantes. Les premiers résultats (voir figure 1) montrent qu'un des peptides modifiés, à savoir l'analogue E (IS de 7.7 à 90 µM) a une activité analogue, voir supérieure selon les expériences, à celle du peptide parent (IS de 6.7 à 90 µM).

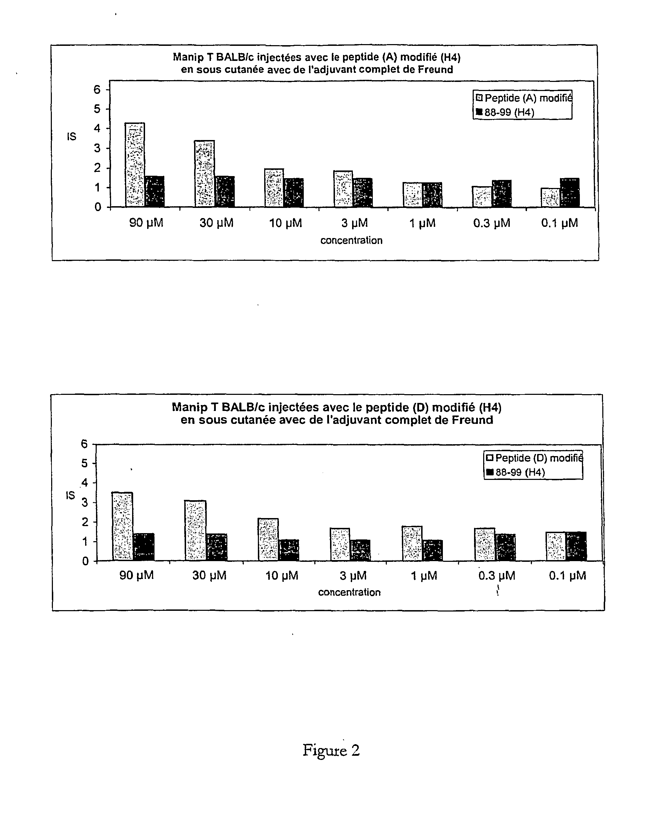

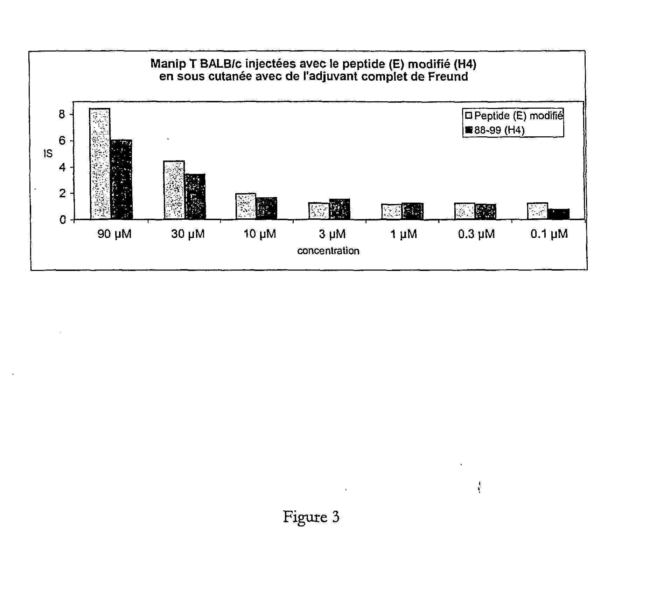

Par ailleurs une manip miroir de la précédente a été réalisée, à savoir des tests de réponse sur les lymphocytes T de souris injectées avec les peptides modifiés et stimulation ex vivo avec le peptide parent (88-99). Après injection des analogues, des analyses ont été réalisées sur des cultures de cellules avec lesdits analogues et le peptide parent 88-99. On observe les résultats suivants :
1) les peptides A et D sont immunogènes (induisent des réponses contre le peptide homologue) mais sans réaction croisée avec le peptide parent (voir figure 2).
2) le peptide E est immunogène et les cellules T générées réagissent par réaction croisée avec le peptide parent (voir figure 3).

Le peptide E croise parfaitement avec le peptide parent 88-99.

Les mêmes expériences ont été effectuées avec le peptide G, (correspondant à la séquence SEQ ID NO : 7).

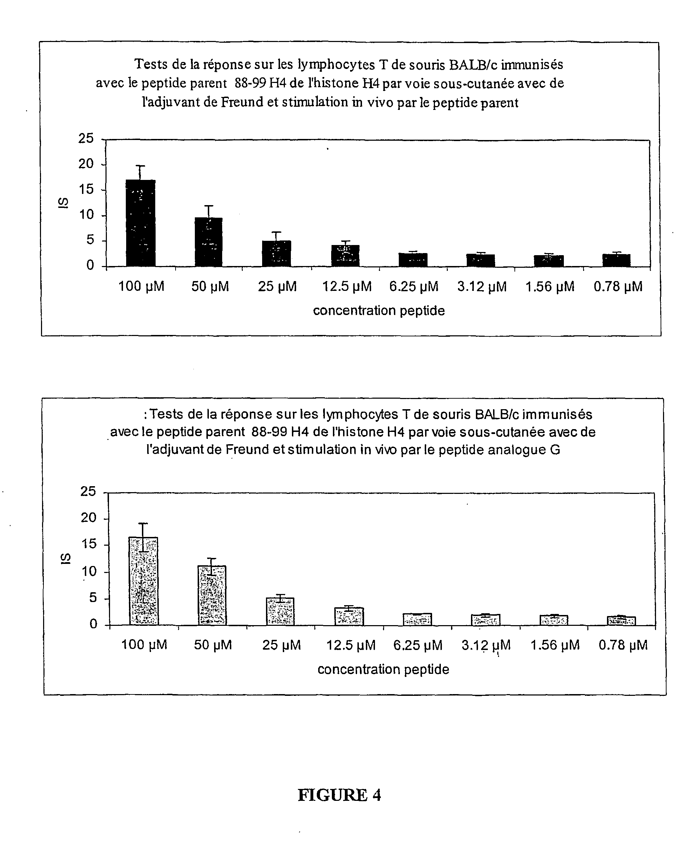

Les résultats (voir Figure 4) montrent que l'analogue G (IS de 16,6 à 100 µM) a une activité analogue à celle du peptide parent (IS de 16,9 à 100 µM).

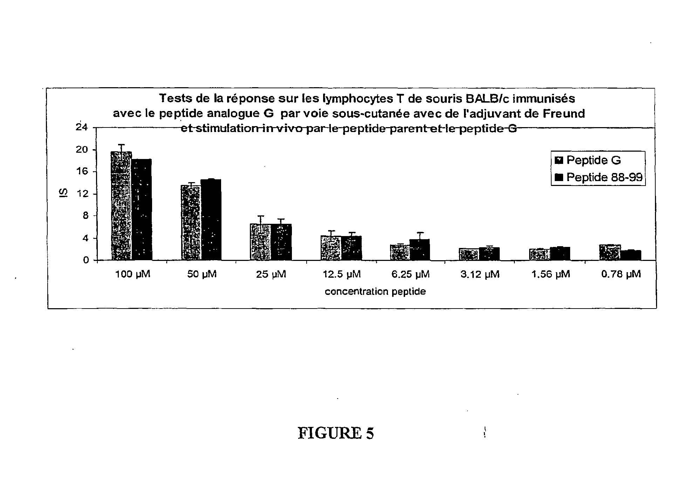

Par ailleurs, une expérience miroir de la précédente a été réalisée, à savoir expérience sur des souris injectées avec le peptide G et stimulation *ex vivo* avec des quantités croissantes du peptide parent (88-99). On observe que le peptide G est immunogène et que les cellules T générées contre ce peptide réagissent par réaction croisée avec le peptide homologue G et surtout avec le peptide parent avec la même intensité (voir Figure 5).

Légende des figures :
- Figure 1 : Tests de réponse sur les lymphocytes T de souris BALB/c injectées avec le peptide parent 88-99 de l'histone H4 en sous cutanée avec de l'adjuvant de Freund, et stimulation ex-vivo avec le peptide parent ou l'analogue E ; L'indice de stimulation est indiqué en ordonnée, et les différentes concentrations en peptide sont indiquées en abscisse.
- Figure 2 : Tests de réponse sur les lymphocytes T de souris BALB/c injectées avec le peptide modifié 88-99 A ou avec le peptide modifié 88-99 D de l'histone H4 en sous cutanée avec de l'adjuvant de Freund. L'indice de stimulation est indiqué en ordonnée, et les différentes concentrations en peptide sont indiquées en abscisse.
- Figure 3 : Tests de réponse sur les lymphocytes T de souris BALB/c injectées avec le peptide modifié 88-99 E de l'histone H4 en sous cutanée avec de l'adjuvant de Freund. L'indice de stimulation est indiqué en ordonnée, et les différentes concentrations en peptide sont indiquées en abscisse.
- Figure 4 : Tests de réponse sur les lymphocytes T de souris BALB/c injectées avec le peptide parent 88-99 de l'histone H4 en sous cutanée avec de l'adjuvant de Freund, et stimulation *ex vivo* avec le peptide parent ou l'analogue G. L'indice de stimulation est indiqué en ordonnée, et les différentes concentrations en peptide sont indiquées en abscisse.
- Figure 5 : Tests de réponse sur les lymphocytes T de souris BALB/c injectées avec le peptide modifié 88-99 G de l'histone H4 en sous cutanée avec de l'adjuvant de Freund. L'indice de stimulation est indiqué en ordonnée, et les différentes concentrations en peptide sont indiquées en abscisse.

### BIBLIOGRAPHIE

Appella, E.; Loftus, D.J.; Sakaguchi, K.; Celis, E. Biomed. Pept. Proteins Nucleic Acids 1996, 1,177.
Mézière, C.; Viguier, M.; Dumortier, H.; Lo-Man, R.; Leclerc, C.; Guillet, J.G.; Briand, J.P.; Muller, S. J. Immunol. 1997, 3230-3237.
Briand, J.P.; Benkirane, N.; Guichard, G.; Newman, J.F.E.; Van Regenmortel, M.H.V.; Brown, F.; Muller, S. Proc. Natl. Acad. Sci. U.S.A. 1997, 94, 12545-12550.
Liu, M.A. Nat. Med., 1998, 4, Suppl. 5, 503.
Stemmer, C.; and Guichard, G. Exp. Opin. Ther. Patents 1998, 8 , 819-830.
Ostankovitch, M, Guichard, G, Connan, F, Muller, S, Chaboissier, A, Hoebeke, J, Choppin, J, Briand, JP, Guillet, JG. J Immunol 1998; 161:200-8.
Petit, M.C.; Benkirane, N.; Guichard, G.; Phan Chan Du, A.; Marraud, M.; Cung, M.T.; Briand, J.P.; Muller, S. J. Biol. Chem. 1999, 274, 3686-3692.
Stemmer, C.; Quesnel, A.; Prévost-Blondel, A.; Zimmermann, C.; Muller, S.; Briand, J.P.; Pircher, H. J. Biol. Chem. 1999, 274, 5550-5555
Ben Yedidia, T., Beignon, AS, Partidos, CD, Muller, S. and Amon, A. Mol. Immunol. 2002, 39, 323-331.
Phan-Chan Du, A., Limal, D., Semetey, V., Dali, H., Jolivet, M., Desgranges, C., Cung, MT, Briand, JP, Petit, MC and Muller, S. J. Mol Biol. 2002, 323, 503-521.
Decker, P., Le Moal, A. , Briand, J.P., Muller. S. J. Immunol. 2000, 165, 654-662.

## Revendications

1. Utilisation de peptides hybrides analogues de peptides ou protéines parents, ces peptides hybrides comprenant au moins un résidu aza-β³ aminoacyle, à savoir :
* un résidu répondant à la formule (A) suivante lorsqu'il est situé en position N-terminale, 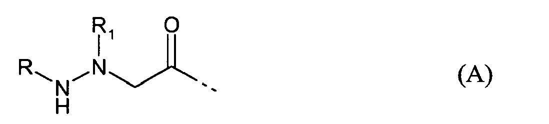
dans laquelle R représente H ou un groupe protecteur de la fonction amine des aminoacides, tels que Fmoc, Boc, ou Z, et R₁ représente une chaîne latérale choisie parmi celles des aminoacides,
* un résidu répondant à la formule (B) suivante lorsqu'il est situé en position C-terminale, 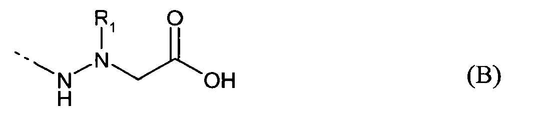
dans laquelle R₁ représente une chaîne latérale choisie parmi celles des aminoacides,
* un résidu répondant à la formule (C) suivante lorsqu'il est situé dans la chaîne desdits peptides hybrides, 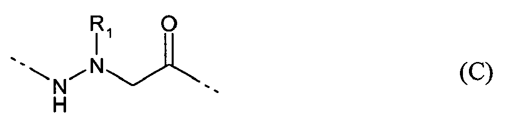 dans laquelle R₁ représente une chaîne latérale choisie parmi celles des aminoacides,
lesdits peptides hybrides étant **caractérisés en ce qu'**ils sont issus de l'épitope 88-99 de l'histone H4 à titre de peptide parent, correspondant à SEQ ID NO : 1, dont l'un au moins des aminoacides initiaux est substitué par un résidu analogue aza-β³ aminoacide, pour la préparation d'un médicament, ou vaccin, destiné à la prévention ou au traitement du lupus érythémateux disséminé.

2. Utilisation selon la revendication 1, de peptides hybrides de formule (I) suivante :
(I) aa₁-N^{α}haaₘ-aaₙ-N^{α}haaₒ-aaₚ
dans laquelle :
- aa₁, aaₙ et aaₚ représentent un résidu aminoacyle, ou un enchaînement de résidus aminoacyles, correspondant aux résidus aminoacyles présents aux mêmes positions dans le peptide ou la protéine parent dont les peptides hybrides sont issus,
- N^{α}haaₘ et N^{α}haaₒ représentent un résidu monomère aza-β³aminoacyle, ou un enchaînement de résidus monomères aza-β³ aminoacyles, analogues aux résidus aminoacyles initialement présents à la même position dans le peptide ou la protéine parent dont les peptides hybrides sont issus, lesdits monomères aza-β³ aminoacyles répondant aux formules (A), (B), ou (C) indiquées dans la revendication 1, suivant qu'ils soient respectivement en position N-terminale, C-terminale, ou dans la chaîne desdits peptides hybrides, et dans lesquelles R₁ est identique à la chaîne latérale de l'aminoacide initial du peptide ou de la protéine parent auquel correspondent lesdits monomères aza-β³ aminoacyles,
- 1, m, n, o et p représentent zéro, ou un nombre entier compris entre 1 et 20, sous réserve que l'un au moins de m ou de o soit différent de zéro, et que le nombre minimum de résidus dans lesdits peptides hybrides de formule (I) soit de 4.

3. Utilisation selon la revendication 1, de peptides hybrides de formules suivantes :
- SEQ ID NO : 2 (ou peptide E) :
⁸⁸H₂N- Tyr-Ala-Leu-Lys-Arg-Gln-Gly-Arg- Thr-**N^{α}**-**hLeu**- Tyr-Gly-OH⁹⁹
- SEQ ID NO : 3 (ou peptide C) :
⁸⁸H₂N-Tyr-Ala-**N**^{α}-**hLeu**- Lys-Arg-Gln-Gly-Arg- Thr-Leu- Tyr-G ly-OH⁹⁹
- SEQ ID NO : 4 (ou peptide A) :
⁸⁸H₂N- Tyr-**N^{α}-hAla**-Leu-Lys-Arg-Gln-Gly-Arg- Thr-Leu- Tyr-Gly-OH⁹⁹
- SEQ ID NO : 5 (ou peptide B) :
⁸⁸H₂N-Tyr-**N^{α}-hAla-N^{α}-hLeu**-Lys-Arg-Gln-Gly-Arg-Thr-Leu-Tyr-Gly-OH⁹⁹
- SEQ ID NO : 6 (ou peptide D) :
⁸⁸H₂N-Tyr-Ala-Leu-**N^{α}-hLys-Arg**-Gln-Gly-Arg-Thr-Leu-Tyr-Gly-OH⁹⁹
- SEQ ID NO : 7 (ou peptide G) :
⁸⁸H₂N-Tyr-Ala-Leu-Lys-Arg-Gln-Gly-Arg-Thr-**N^{α}**-**hLeu**-**N^{α}-hTyr**-Gly-OH⁹⁹
- SEQ ID NO : 8 :
⁸⁸H₂N-Tyr-Ala-Leu-Lys-Arg-Gln-**N^{α}-hGly**-Arg-Thr-Leu-Tyr-Gly-OH⁹⁹
- SEQ ID NO : 9 :
⁸⁸H₂N-Tyr-Ala-Leu-Lys-Arg-Gln-Gly-**N^{α}-hArg**-Thr-Leu-Tyr-Gly-OH⁹⁹
- SEQ ID NO : 10 :
⁸⁸H₂N-Tyr-Ala-Leu-Lys-**N^{α}-hArg-**Gln-Gly-Arg-Thr-Leu-Tyr-Gly-OH⁹⁹
- SEQ ID NO : 11 :
⁸⁸H₂N-Tyr-Ala-Leu-Lys-Arg-Gln-Gly-Arg-Thr-Leu-**N^{α}-hTyr**-Gly-OH⁹⁹
- SEQ ID NO : 12 :
⁸⁸H₂N-**N^{α}-hTyr**-Ala-Leu-Lys-Arg-Gln-Gly-Arg-Thr-Leu-Tyr-Gly-OH⁹⁹
- SEQ ID NO : 13 :
⁸⁸HN-Tyr-Ala-Leu-Lys-Arg-Gln-Gly-Arg-Thr-Leu-**Tyr-N^{α}-hGly**-OH⁹⁹
- SEQ ID NO:14:
⁸⁸H₂N-Tyr-Ala-Leu-Lys-Arg-Gln-Gly-Arg-Thr-**N^{α}-hLeu-N^{α}-hTyr-N^{α}-hGly**-OH⁹⁹

4. Utilisation selon la revendication 3, du peptide hybride de formule SEQ ID NO : 2, ou du peptide hybride de formule SEQ ID NO : 7.

5. Peptides hybrides comprenant au moins un aza-β³ aminoacide, ces peptides hybrides étant des analogues de peptides ou protéines parents, lesdits peptides hybrides comprenant au moins un aminoacide initial du peptide ou de la protéine parent, et étant issus de l'épitope 88-99 de l'histone H4 à titre de peptide parent, correspondant à SEQ ID NO : 1.

6. Peptides hybrides selon la revendication 5, de formule (I) suivante :
(I) aa₁-N^{α}haaₘ-aaₙ-N^{α}haaₒ-aaₚ
dans laquelle :
- aa₁, aaₙ et aaₚ représentent un résidu aminoacyle, ou un enchaînement de résidus aminoacyles, correspondant aux résidus aminoacyles présents aux mêmes positions dans le peptide ou la protéine parent dont les peptides hybrides sont issus,
- N^{α}haaₘ et N^{α}haaₒ représentent un résidu monomère aza-β³ aminoacyle, ou un enchaînement de résidus monomères aza-β³ aminoacyles, analogues aux résidus aminoacyles initialement présents à la même position dans le peptide ou la protéine parent dont les peptides hybrides sont issus, lesdits monomères aza-β³ aminoacyles répondant aux formules (A), (B), ou (C) indiquées dans la revendication 1, suivant qu'ils soient respectivement en position N-terminale, C-terminale, ou dans la chaîne desdits peptides hybrides, et dans lesquelles R₁ est identique à la chaîne latérale de l'aminoacide initial du peptide ou de la protéine parent auquel correspondent lesdits monomères aza-β³ aminoacyles,
- 1, m, n, o et p représentent zéro, ou un nombre entier compris entre 1 et 20, sous réserve que l'un au moins de m ou de o soit différent de zéro, que le nombre minimum de résidus dans lesdits peptides hybrides de formule (I) soit de 4, et l'un au moins de 1, n, ou p soit différent de zéro.

7. Peptides hybrides selon la revendication 5 ou 6, de formules suivantes :
- SEQ ID NO : 2 (ou peptide E) :
⁸⁸H₂N-Tyr-Ala-Leu-Lys-Arg-Gln-Gly-Arg-Thr-**N^{α}-hLeu-**Tyr-Gly-OH⁹⁹
- SEQ ID NO : 3 (ou peptide C) :
⁸⁸H₂N-Tyr-Ala-**N^{α}-hLeu**-Lys-Arg-Gln-Gly-Arg-Thr-Leu-Tyr-Gly-OH⁹⁹
- SEQ ID NO : 4 (ou peptide A) :
⁸⁸H₂N-Tyr-**N^{α}-hAla-**Leu-Lys-Arg-Gln-Gly-Arg-Thr-Leu-Tyr-Gly-OH⁹⁹
- SEQ ID NO : 5 (ou peptide B) :
⁸⁸H₂N-Tyr-**N^{α}-hAla-N^{α}-hLeu**-Lys-Arg-Gln-Gly-Arg-Thr-Leu-Tyr-Gly-OH⁹⁹
- SEQ ID NO : 6 (ou peptide D) :
⁸⁸H₂N- Tyr-Ala-Leu-**N^{α}-hLys**-Arg-Gln-Gly-Arg- Thr-Leu- Tyr-Gly-OH⁹⁹
- SEQ ID NO : 7 (ou peptide G) :
⁸⁸H₂N-Tyr-Ala-Leu-Lys-Arg-Gln-Gly-Arg-Thr-**N^{α}-hLeu-N^{α}-hTyr**-Gly-OH⁹⁹
- SEQ ID NO : 8 :
⁸⁸H₂N-Tyr-Ala-Leu-Lys-Arg-Gln-**N^{α}-hGly**-Arg-Thr-Leu-Tyr-Gly-OH⁹⁹
- SEQ ID NO : 9 :
⁸⁸H₂N-Tyr-Ala-Leu-Lys-Arg-Gln-Gly-**N^{α}-hArg**-Thr-Leu-Tyr-Gly-OH⁹⁹
- SEQ ID NO : 10 :
⁸⁸H₂N-Tyr-Ala-Leu-Lys-**N^{α}-hArg**-Gln-Gly-Arg-Thr-Leu-Tyr-Gly-OH⁹⁹
- SEQ ID NO : 11 :
⁸⁸H₂N-Tyr-Ala-Leu-Lys-Arg-Gln-Gly-Arg-Thr-Leu-**N^{α}-hTyr**-Gly-OH⁹⁹
- SEQ ID NO : 12 (ou peptide F):
⁸⁸H₂N-**N^{α}**-**hTyr**-Ala-Leu-Lys-Arg-Gln-Gly-Arg-Thr-Leu-Tyr-Gly-OH⁹⁹
- SEQ ID NO : 13 (ou peptide H):
⁸⁸H₂N- Tyr-Ala-Leu-Lys-Arg-Gln-Gly-Arg- Thr-Leu-Tyr-**N^{α}-hGly**-OH⁹⁹
- SEQ ID NO : 14 (ou peptide I) :
⁸⁸H₂N-Tyr-Ala-Leu-Lys-Arg-Gln-Gly-Arg-Thr-**N^{α}-hLeu-N^{α}-hTyr-N^{α}-hGly**-OH⁹⁹

8. Peptides hybrides selon l'une des revendications 5 à 7, de formules suivantes :
- SEQ ID NO : 2 (ou peptide E):
⁸⁸H₂N-Tyr-Ala-Leu-Lys-Arg-Gln-Gly-Arg-Thr-**N^{α}-hLeu**-Tyr-Gly-OH⁹⁹
- SEQ ID NO : 7 (ou peptide G) :
⁸⁸H₂N-Tyr-Ala-Leu-Lys-Arg-Gln-Gly-Arg- Thr-**N^{α}-hLeu-N^{α}-hTyr**-Gly-OH⁹⁹

9. Anticorps anti-peptides hybrides polyclonaux ou monoclonaux tels qu'obtenus par immunisation d'un animal avec au moins un peptide hybride défini dans l'une des revendications 1 à 8, lesdits anticorps étant susceptibles de former un complexe avec ces peptides hybrides, et/ou avec les peptides ou protéines parents correspondant à ces derniers, et **caractérisés en ce qu'**ils reconnaissent le peptide parent ou la protéine parente avec une affinité au moins égale à celle présentée par les anticorps anti-peptide parent ou anti-protéine parente vis à vis du peptide parent ou de la protéine parente.

10. Anticorps anti-idiotypes susceptibles de former un complexe avec les anticorps selon la revendication 9, tels qu'obtenus par immunisation d'un animal avec lesdits anticorps selon la revendication 9.

11. Complexe entre un peptide hybride tel que défini dans l'une des revendications 1 à 8, et un élément du complexe d'histocompatibilité majeur (encore désigné complexe MHC-hybride), et éventuellement un récepteur de cellules T (encore désigné complexe MHC-hybride-récepteur T).

12. Complexe entre un peptide hybride tel que défini dans l'une des revendications 1 à 8, et un récepteur de cellules T.

13. Méthode de diagnostic *in vitro* de pathologies associées à la présence dans l'organisme d'un patient, d'une protéine exogène ou endogène, susceptible d'être directement ou indirectement impliquée dans le processus d'apparition et/ou de développement de ces pathologies, **caractérisée en ce qu'**elle comprend :
- la mise en contact d'un échantillon biologique provenant d'un patient susceptible d'être porteur d'anticorps dirigés contre ladite protéine, avec un peptide hybride tel que défini dans l'une des revendications 1 à 8, ledit peptide hybride étant issu de tout ou partie de ladite protéine endogène ou exogène, ou issu d'un peptide susceptible d'être reconnu par des anticorps reconnaissant eux-mêmes la protéine exogène ou endogène,
dans des conditions permettant la réaction entre les anticorps dirigés contre la protéine et susceptibles d'être présents dans l'échantillon biologique, et le susdit peptide hybride,
- la détection *in vitro* du complexe antigène / anticorps susceptible d'être formé à l'étape précédente ou
- la détection *in vitro* d'anticorps circulants chez le patient par un test de compétition en utilisant un anticorps anti-hybride.

14. Méthode de diagnostic *in vitro* de pathologies associées à la présence dans l'organisme d'un patient d'une protéine exogène ou endogène, susceptible d'être directement ou indirectement impliquée dans le processus d'apparition et/ou de développement de ces pathologies, ladite méthode étant **caractérisée en ce qu'**elle comprend :
- la mise en contact d'un échantillon biologique provenant d'un patient susceptible d'être porteur de ladite protéine, avec l'un au moins des anticorps selon la revendication 9, les anticorps étant avantageusement dirigés contre un peptide hybride issu de tout ou partie de ladite protéine endogène ou exogène, ou
dans des conditions permettant la réaction entre la protéine susceptible d'être présente dans l'échantillon biologique, et les susdits anticorps dirigés contre le susdit peptide hybride,
- la détection *in vitro* du complexe antigène / anticorps susceptible d'être formé à l'étape précédente, ou
- la détection d'antigènes circulants chez le patient dans des tests de compétition en utilisant un peptide hybride tel que défini dans l'une des revendications 1 à 8.

15. Nécessaire ou kit pour la mise en oeuvre de méthodes de diagnostic *in vitro* selon la revendication 13 ou 14, comprenant :
- un peptide hybride issu de tout ou partie de la protéine endogène ou exogène, ou correspondant à un peptide susceptible d'être reconnu par des anticorps reconnaissant eux-mêmes la protéine exogène ou endogène, ou bien des anticorps selon la revendication 9, dirigés contre ce peptide hybride ;
- des réactifs pour rendre un milieu apte à la formation d'une réaction immunologique ;
- des réactifs permettant de détecter le complexe antigène / anticorps qui a été produit à l'issue de la réaction immunologique, lesdits réactifs contenant éventuellement un marqueur ou étant susceptibles d'être reconnus à leur tour par un réactif marqué, plus particulièrement dans le cas où le peptide hybride ou les anticorps anti-hybrides susmentionnés ne sont pas marqués.

16. Composition pharmaceutique, notamment vaccin, **caractérisée en ce qu'**elle comprend un peptide hybride tel que défini dans l'une des revendications 1 à 8, ou un anti-idiotype selon la revendication 10, en association ou non avec un véhicule physiologiquement acceptable.

17. Composition pharmaceutique **caractérisée en ce qu'**elle comprend un peptide hybride tel que défini dans l'une des revendications 1 à 8, ou un anticorps anti-idiotype selon la revendication 10, associés à une molécule porteuse, protéique ou non, pouvant induire *in vivo* la production d'anticorps neutralisant la protéine exogène ou endogène responsable de la pathologie, ou induire *in vivo* une réponse immune cellulaire cytotoxique ou auxiliaire.

18. Composition pharmaceutique, **caractérisée en ce qu'**elle comprend des anticorps selon la revendication 9, en association avec un véhicule physiologiquement acceptable.

## Claims

1. Use of hybrid peptides analogues of parent peptides or proteins, these hybrid peptides containing at least one aza-β³ aminoacyl residue, namely :
* a residue corresponding to the following formula (A) when it is situated in the N-terminal position, 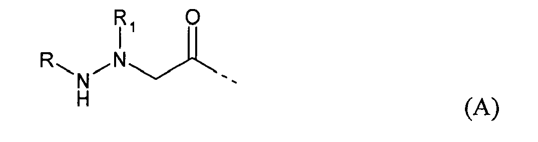
wherein R represents H or a protecting group of the amine function of the amino acids, such as Fmoc, Boc, or Z, and R₁ represents a side-chain selected from those of the amino acids,
* a residue corresponding to the following formula (B) when it is situated in the C-terminal position, 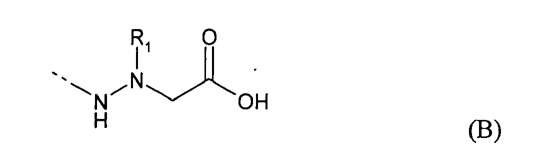
wherein R₁ represents a side-chain selected from those of the amino acids,
* a residue corresponding to the following formula (C) when it is situated inside the chain of the said hybrid peptides, 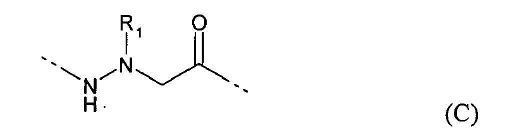 wherein R₁ represents a side-chain selected from those of the amino acids,
said hybrid peptides being **characterized in that** they derive from the epitope 88-99 of the histone H4 as parent peptide, and corresponding to SEQ ID NO : 1, wherein at least one of the initial amino acids is replaced by an aza-β³ amino acid analogue residue,
for the preparation of a drug, or a vaccine, for the prevention or the treatment of systemic lupus erythematosus.

2. Use according to claim 1, of hybrid peptides of the following formula (I):
(I) aa₁-N^{α}haaₘ-aaₙ-N^{α}haaₒ-aaₚ
wherein :
- aa₁, aaₙ and aaₚ represent an aminoacyl residue, or a chain of aminoacyl residues, corresponding to the aminoacyl residues present at the same positions in the parent peptide or protein from which the hybrid peptides are derived,
- N^{α}haaₘ and N^{α}haaₒ represent an aza-β³ aminoacyl monomer residue, or a chain of aza-β³ aminoacyl monomer residues, analogous to the aminoacyl residues initially present at the same position in the parent peptide or protein from which the hybrid peptides are derived, the said aza-β³ aminoacyl monomers corresponding to the formulae (A), (B), or (C) shown in claim 1, depending on whether they are respectively in the N-terminal or C-terminal position, or inside the chain of the said hybrid peptides, and wherein R₁ is identical to the side-chain of the initial amino acid of the parent peptide or protein to which the said aza-β³ aminoacyl monomers correspond,
- l, m, n, o, and p represent zero, or a whole number comprised between 1 and 20, provided that at least one of m or o is different from zero, and that the minimum number of residues in the said hybrid peptides of formula (I) is 4.

3. Use according to claim 1, of hybrid peptides of the following formulae:
- SEQ ID NO : 2 (or peptide E) :
⁸⁸H₂N-Tyr-Ala-Leu-Lys-Arg-Gln-Gly-Arg-Thr-**N^{α}-hLeu**-Tyr-Gly-OH⁹⁹
- SEQ ID NO : 3 (or peptide C) :
⁸⁸H₂N-Tyr-Ala-**N^{α}-hLeu**-Lys-Arg-Gln-Gly-Arg-Thr-Leu-Tyr-Gly-OH⁹⁹
- SEQ ID NO : 4 (or peptide A) :
⁸⁸H₂N-Tyr-**N^{α}-hAla**-Leu-Lys-Arg-Gln-Gly-Arg-Thr-Leu-Tyr-Gly-OH⁹⁹
- SEQ ID NO : 5 (or peptide B) :
⁸⁸H₂N-Tyr-**N^{α}-hAla-N^{α}-hLeu**-Lys-Arg-Gln-Gly-Arg-Thr-Leu-Tyr-Gly-OH⁹⁹
- SEQ ID NO : 6 (or peptide D) :
⁸⁸H₂N-Tyr-Ala-Leu-**N^{α}-hLys**-Arg-Gln-Gly-Arg-Thr-Leu-Tyr-Gly-OH⁹⁹
- SEQ ID NO : 7 **(or peptide G) :**
⁸⁸H₂N-Tyr-Ala-Leu-Lys-Arg-Gln-Gly-Arg-Thr-**N^{α}-hLeu-N^{α}-hTyr**-Gly-OH⁹⁹
- SEQ ID NO : 8 :
⁸⁸H₂N-Tyr-Ala-Leu-Lys-Arg-Gln-**N^{α}**-**hGly**-Arg-Thr-Leu-Tyr-Gly-OH⁹⁹
- SEQ ID NO : 9 :
⁸⁸H₂N-Tyr-Ala-Leu-Lys-Arg-Gln-Gly-**N^{α}-hArg**-Thr-Leu-Tyr-Gly-OH⁹⁹
- SEQ ID NO : 10 :
⁸⁸H₂N-Tyr-Ala-Leu-Lys-**N^{α}-hArg**-Gln-Gly-Arg-Thr-Leu-Tyr-Gly-OH⁹⁹
- SEQ ID NO : 11 :
⁸⁸H₂N-Tyr-Ala-Leu-Lys-Arg-Gln-Gly-Arg-Thr-Leu-**N^{α}-hTyr**-Gly-OH⁹⁹
- SEQ ID NO : 12:
⁸⁸H₂N-**N^{α}-hTyr**-Ala-Leu-Lys-Arg-Gln-Gly-Arg-Thr-Leu-Tyr-Gly-OH⁹⁹
- SEQ ID NO : 13 :
⁸⁸H₂N-Tyr-Ala-Leu-Lys-Arg-Gln-Gly-Arg-Thr-Leu-Tyr-**N^{α}**-**hGly**-OH⁹⁹
- SEQ ID NO : 14 :
⁸⁸H₂N-Tyr-Ala-Leu-Lys-Arg-Gln-Gly-Arg-Thr-**N^{α}-hLeu-N**^{α}**-hTyr-N-hGl**y-OH⁹⁹

4. Use according to claim 3, of the hydrid peptide of the formula SEQ ID NO : 2, or of the hybrid peptide of the formula SEQ ID NO : 7.

5. Hybrid peptides containing at least one aza-β³ amino acid, these hybrid peptides being analogues of parent peptides or proteins, the said hybrid peptides containing at least one initial amino acid of the parent peptide or protein, and deriving from the epitope 88-99 of the histone H4 as parent peptide, corresponding to SEQ ID NO : 1.

6. Hybrid peptides according to claim 5, of the following formula (I):
(I) aa₁-N^{α}haaₘ-aaₙ-N^{α}haaₒ-aaₚ
wherein :
- aa₁, aaₙ and aaₚ represent an aminoacyl residue, or a chain of aminoacyl residues, corresponding to the aminoacyl residues present at the same positions in the parent peptide or protein from which the hybrid peptides are derived,
- N^{α}haaₘ and N^{α}haaₒ represent an aza-β³ aminoacyl monomer residue, or a chain of aza-β³ aminoacyl monomer residues analogous to the aminoacyl residues initially present at the same position in the parent peptide or protein from which the hybrid peptides are derived, the said aza-β³ aminoacyl monomers corresponding to the formulae (A), (B), or (C) shown in claim 1, depending on whether they are respectively in the N-terminal or C-terminal position, or inside the chain of the said hybrid peptides, and wherein R₁ is identical to the side-chain of the initial amino acid of the parent peptide or protein to which the said aza-β³ aminoacyl monomers correspond,
- l, m, n, o, and p represent zero, or a whole number comprised between 1 and 20, provided that at least one of m or o is different from zero, that the minimum number of residues in the said hybrid peptides of formula (I) is 4, and that at least one of 1, n, or p is different from zero.

7. Hybrid peptides according to claims 5 or 6, of the following formulae:
- SEQ ID NO : 2 (or peptide E) :
⁸⁸H₂N- Tyr-Ala-Leu-Lys-Arg-Gln-Gly-Arg- Thr-**N^{α}-hLeu**-Tyr-Gly-OH⁹⁹
- SEQ ID NO : 3 (or peptide C) :
⁸⁸H₂N-Tyr-Ala-**N^{α}-hLeu**-Lys-Arg-Gln-Gly-Arg-Thr-Leu-Tyr-Gly-OH⁹⁹
- SEQ ID NO : 4 (or peptide A) :
⁸⁸ H₂N-Tyr-**N^{α}-hAla**-Leu-Lys-Arg-Gln-Gly-Arg-Thr-Leu-Tyr-Gly-OH⁹⁹
- SEQ ID NO : 5 (or peptide B) :
⁸⁸H₂N-Tyr-**N^{α}-hAla-N^{α}-hLeu**-Lys-Arg-Gln-Gly-Arg-Thr-Leu-Tyr-Gly-OH⁹⁹
- SEQ ID NO : 6 (or peptide D) :
⁸⁸H₂N- Tyr-Ala-Leu-**N^{α}-hLys**-Arg-Gln-Gly-Arg- Thr-Leu- Tyr-Gly-OH⁹⁹
- SEQ ID NO : 7 **(or peptide G) :**
⁸⁸H₂N- Tyr-Ala-Leu-Lys-Arg-Gln-Gly-Arg- Thr-**N^{α}-hLeu**-**N^{α}-hTyr**-Gly-OH⁹⁹
- SEQ ID NO : 8 :
⁸⁸H₂N-Tyr-Ala-Leu-Lys-Arg-Gln-**N^{α}**-**hGly**-Arg-Thr-Leu-Tyr-Gly-OH⁹⁹
- SEQ ID NO : 9 :
⁸⁸H₂N-Tyr-Ala-Leu-Lys-Arg-Gln-Gly-**N^{α}-hArg**-Thr-Leu-Tyr-Gly-OH⁹⁹
- SEQ ID NO: 10 :
⁸⁸H₂N-Tyr-Ala-Leu-Lys-**N^{α}-hArg**-Gln-Gly-Arg-Thr-Leu-Tyr-Gly-OH⁹⁹
- SEQ ID NO : 11 :
⁸⁸H₂N-Tyr-Ala-Leu-Lys-Arg-Gln-Gly-Arg-Thr-Leu-**N^{α}-hTyr**-Gly-OH⁹⁹
- SEQ ID NO: 12:
⁸⁸H₂N-**N^{α}-hTyr**-Ala-Leu-Lys-Arg-Gln-Gly-Arg- Thr-Leu- Tyr-Gly-OH⁹⁹
- SEQ ID NO : 13 :
⁸⁸H₂N-Tyr-Ala-Leu-Lys-Arg-Gln-Gly-Arg-Thr-Leu-Tyr-**N^{α}-hGly**-OH⁹⁹
- SEQ ID NO : 14 :
⁸⁸H₂N-Tyr-Ala-Leu-Lys-Arg-Gln-Gly-Arg-Thr-**N^{α}-hLeu-N^{α}-hTyr-N^{α}-hGly**-OH⁹⁹

8. Hybrid peptides according to any of the claims 5 to 7, of the following formulae:
- SEQ ID NO : 2 (or peptide E) :
⁸⁸H₂N-Tyr-Ala-Leu-Lys-Arg-Gln-Gly-Arg-Thr-**N**^{α}-**hLeu**-Tyr-Gly-OH⁹⁹
- SEQ ID NO : 7 (or peptide G) :
⁸⁸H₂N-Tyr-Ala-Leu-Lys-Arg-Gln-Gly-Arg-Thr-**N^{α}-hLeu**-**N^{α}-hTyr**-Gly-OH⁹⁹

9. Polyclonal or monoclonal anti-hybrid peptide antibodies such as obtained by immunization of an animal with at least one hybrid peptide defined in any of claims 1 to 8, the said antibodies being capable of forming a complex with these hybrid peptides, and/or with the parent peptides or proteins corresponding to these latter, and **characterized in that** they recognize the parent peptide or the parent protein with an affinity at least equal to that displayed by the anti-parent peptide or anti-parent protein antibodies towards the parent peptide or the parent protein.

10. Anti-idiotype antibodies capable of forming a complex with antibodies according to claim 9, such as obtained by immunization of an animal with said antibodies according to claim 9.

11. A complex between a hybrid peptide such as defined in any of claims 1 to 8, and an element of the major histocompatibility complex (also referred to as MHC-hybrid complex), and possibly a T cell receptor (also referred to as MHC-hybrid-T receptor complex).

12. A complex between a hybrid peptide such as defined in any of claims 1 to 8, and a T cell receptor.

13. A method for the *in vitro* diagnosis of pathologies associated with the presence in the body of a patient, of an exogenous or endogenous protein, capable of being directly or indirectly involved in the process of appearance and/or development of these pathologies, **characterized in that** it comprises:
- contacting a biological sample deriving from a patient capable of being a carrier of antibodies directed against the said protein, with a hybrid peptide such as defined in any of claims 1 to 8, the said hybrid peptide being derived from all or part of the said endogenous or exogenous protein, or derived from a peptide capable of being recognized by antibodies themselves recognizing the exogenous or endogenous protein,
under conditions allowing the reaction between the antibodies directed against the protein and capable of being present in the biological sample, and the aforesaid hybrid peptide,
- the *in vitro* detection of the antigen / antibody complex capable of being formed in the preceding stage or
- the *in vitro* detection of antibodies circulating in the patient by a competitive test using an anti-hybrid antibody.

14. A method for the *in vitro* diagnosis of pathologies associated with the presence in the body of a patient of an exogenous or endogenous protein, capable of being directly or indirectly involved in the process of appearance and/or development of these pathologies, the said method being **characterized in that** it comprises:
- contacting a biological sample deriving from a patient capable of being a carrier of the said protein with at least one of the antibodies according to claim 9, said antibodies being advantageously directed against a hybrid peptide derived from all or part of the said endogenous or exogenous protein, or
under conditions allowing the reaction between the protein capable of being present in the biological sample, and the aforesaid antibodies directed against the aforesaid hybrid peptide;
- the *in vitro* detection of the antigen / antibody complex capable of being formed in the preceding stage, or
- the detection of antigens circulating in the patient in competitive tests using a hybrid peptide such as defined in any of claims 1 to 8.

15. A set or kit for the implementation of the *in vitro* diagnostic methods according to claim 13 or 14, comprising:
- a hybrid peptide derived from all or part of the endogenous or exogenous protein, or corresponding to a peptide capable of being recognized by antibodies themselves recognizing the exogenous or endogenous protein, or by antibodies according to claim 9, directed against said hybrid peptide;
- reagents to render a medium suitable for the formation of an immunological reaction,
- reagents making it possible to detect the antigen / antibody complex which has been formed as a result of the immunological reaction, the said reagents possibly containing a marker or being capable of being recognized in their turn by a labeled reagent, more particularly in the case where the hybrid peptide or the aforesaid anti-hybrid antibodies are not labeled.

16. A pharmaceutical composition, in particular vaccine, **characterized in that** it comprises a hybrid peptide such as defined in any of claims 1 to 8, or an anti-idiotype according to claim 10, in association or not with a physiologically acceptable vehicle.

17. A pharmaceutical composition, **characterized in that** it comprises a hybrid peptide such as defined in any of claims 1 to 8, or an anti-idiotype antibody according to claim 10, associated with a carrier molecule, proteic or not, capable of inducing *in vivo* the production of an antibody neutralizing the exogenous or endogenous protein responsible for the pathology, or inducing *in vivo* a cytotoxic or auxiliary cellular immune response.

18. A pharmaceutical composition, **characterized in that** it comprises antibodies according to claim 9, in association with a physiologically acceptable vehicle.

## Patentansprüche

1. Verwendung von Hybridpeptidanaloga von parenten Peptiden oder Proteinen, wobei diese Hybridpeptide mindestens einen Aminoacyl-Aza-Beta-3-Rest enthalten, nämlich:
- einen Rest der der folgenden Formel (A) entspricht, wenn er sich in der N-terminalen Position befindet: 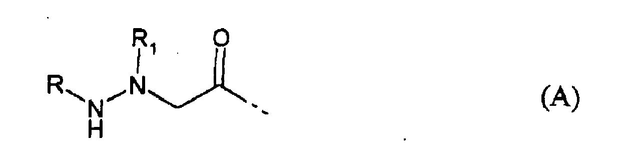
wobei R H oder eine Schutzgruppe der Aminfunktion der Aminosäuren, wie beispielsweise Fmoc, Boc oder Z darstellt, und R₁ eine seitliche Kette darstellt, die unter jenen der Aminosäuren ausgewählt wird,
- einen Rest, der der folgenden Formel (B) entspricht, wenn er sich in der C-terminalen Position befindet: 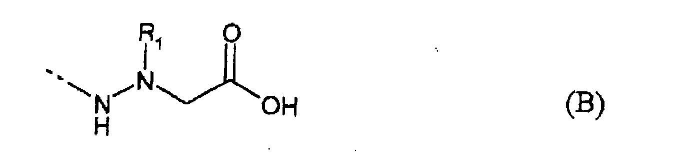
wobei R₁ eine seitliche Kette darstellt, die unter jenen der Aminosäuren ausgewählt wird,
- einen Rest, der der folgenden Formel (C) entspricht, wenn er sich in der Kette der Hybridpeptide befindet: 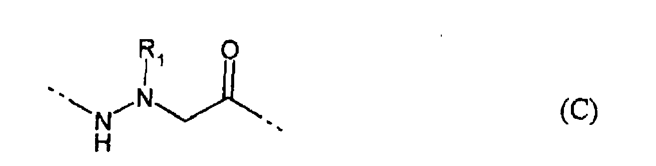 wobei R₁ eine seitliche Kette darstellt, die unter jenen der Aminosäuren ausgewählt wird,
wobei die Hybridpeptide **dadurch gekennzeichnet sind, dass** sie vom Epitop 88-99 des Histons H4 als parentes Peptid entsprechend SEQ ID NO: 1 stammen, wobei mindestens eine der ursprünglichen Aminosäuren durch einen Aminoacyl-Aza-Beta-3-Analogrest ersetzt wird, um ein Medikament oder einen Impfstoff herzustellen, der zur Prävention oder Behandlung von verstreutem Lupus erythematodes bestimmt ist.

2. Verwendung nach Anspruch 1 von Hybridpeptiden mit folgender Formel (I):
(I) aa₁-N^{α}haaₘ-aaₙ-N^{α}haaₒ-aaₚ
wobei
- aa₁, aaₙ und aaₚ einen Aminoacylrest oder eine Verkettung von Aminoacylresten darstellen, die den Aminoacylresten entsprechen, die an denselben Positionen im Peptid oder parenten Protein, von denen die Hybridpeptide ausgegangen sind, vorhanden sind,
- N^{α}haaₘ und N^{α}haaₒ einen Monomer-Aminoacyl-Aza-Beta-3-Rest oder eine Verkettung von Monomer-Aminoacyl-Aza-Beta-3-Resten analog zu den Aminoacylresten, die ursprünglich an derselben Position im Peptid oder parenten Protein vorhanden waren, von denen die Hybridpeptide ausgegangen sind, wobei die die Aminoacyl-Aza-Beta-3-Monomere den Formeln (A), (B) oder (C), die in Anspruch 1 angeführt sind, entsprechen, je nachdem, ob sie sich an der N-terminalen, C-terminalen Position oder in der Kette der Hybridpeptide befinden, und bei denen R₁ mit seitlichen Kette der ursprünglichen Aminosäure des Peptids oder des parenten Proteins, dem die Aminoacyl-Aza-Beta-3-Monomere entsprechen, identisch ist,
- l, m, n, o und p Null oder eine ganze Zahl zwischen 1 und 20 darstellen, vorbehaltlich dessen, dass mindestens m oder o ungleich Null ist, und dass die Mindestzahl von Resten in den Hybridpeptiden mit der Formel (I) gleich 4 ist.

3. Verwendung nach Anspruch 1 von Hybridpeptiden mit folgenden Formeln:
- SEQ ID NO : 2 (oder Peptid E) :
⁸⁸H2N-Tyr-Ala-Leu-Lys-Arg-Gln-Gly-Arg- Thr-**N^{α} -hLeu**-Tyr-Gly-OH⁹⁹
SEQ ID NO: 3 (oder Peptid C) :
⁸⁸H₂N- Tyr-Ala-**N^{α}-hLeu**-Lys-Arg-Gln-Gly-Arg-Thr-Leu-Tyr-Gly-OH⁹⁹
SEQ ID NO : 4 (oder Peptid A) :
⁸⁸H₂N-Tyr-**N^{α}-hAla**-Leu-Lys-Arg-Gln-Gly-Arg-Thr-Leu-Tyr-Gly-OH⁹⁹
- SEQ ID NO : 5 (oder Peptid B):
⁸⁸H₂N- Tyr-**N^{α}-hAla-N^{α}-hLeu-**Lys-Arg-Gln-Gly-Arg- Thr-Leu- Tyr-Gly-OH⁹⁹
- SEQ ID NO :6 (oder Peptid D) :
⁸⁸H₂N-Tyr-Ala-Leu-**N^{α}-hLys-**Arg-Gln-Gly-Arg-Thr-Leu-Tyr-Gly-OH⁹⁹
- SEQ ID NO:7 (oder Peptid G):
⁸⁸H₂N-Tyr-Ala-Leu-Lys-Arg-Gln-Gly-Arg-Thr-**N^{α}-hLeu-N^{α}-hTyr**-Gly-OH⁹⁹
- SEQ ID NO : 8 :
⁸⁸H₂N-Tyr-Ala-Leu-Lys-Arg-Gln-**N^{α}-hGly**-Arg-Thr-Leu-Tyr-Gly-OH⁹⁹
- SEQ ID NO: 9:
⁸⁸H₂N-Tyr-Ala-Leu-Lys-Arg-Gln-Gly-**N^{α}-hArg**-Thr-Leu-Tyr-Gly-OH⁹⁹
- SEQ ID NO : 10 :
⁸⁸H₂N-Tyr-Ala-Leu-Lys-**N^{α}-hArg-**Gln-Gly-Arg-Thr-Leu-Tyr-Gly-OH⁹⁹
- SEQ ID NO : 11 :
⁸⁸H₂N-Tyr-Ala-Leu-Lys-Arg-Gln-Gly-Arg-Thr-Leu-**N^{α}-hTyr**-Gly-OH⁹⁹
- SEQ ID NO : 12 :
⁸⁸H₂N-**N^{α}-hTyr**-Ala-Leu-Lys-Arg-Gin-Gly-Arg-Thr-Leu-Tyr-Gly-OH⁹⁹
- SEQ ID NO : 13 :
⁸⁸H₂N-Tyr-Ala-Leu-Lys-Arg-Gln-Gly-Arg-Thr-Leu-Tyr-**N^{α}-hGly**-OH⁹⁹
- SEQ ID NO : 14 :
⁸⁸H₂N-Tyr-Ala-Leu-Lys-Arg-Gln-Gly-Arg-Thr-**N^{α}-hLeu-N^{α}-hTyr-N^{α}-hGly**-OH⁹⁹

4. Verwendung nach Anspruch 3 des Hybridpeptids mit der Formel SEQ ID NO: 2 oder des Hybridpeptids mit der Formel SEQ ID NO: 7.

5. Hybridpeptide, umfassend mindestens eine Aminosäure-Aza-Beta-3, wobei diese Hybridpeptide Analoga zu parenten Peptiden oder Proteinen sind, wobei die Hybridpeptide mindestens eine ursprüngliche Aminosäure des parenten Peptids oder Proteins umfassen und aus dem Epitop 88-99 des Histons H4 als parentes Peptid entsprechend SEQ ID NO: 1 hervorgegangen sind.

6. Hybridpeptide nach Anspruch 5 mit folgender Formel (I) :
(I) aa₁-N^{α}haaₘ-aaₙ-N^{α}haaₒ-aaₚ
wobei
- aa₁, aaₙ und aaₚ einen Aminoacylrest oder eine Verkettung von Aminoacylresten darstellen, die den Aminoacylresten entsprechen, die an denselben Positionen im Peptid oder parenten Protein, von denen die Hybridpeptide ausgegangen sind, vorhanden sind,
- N^{α}haaₘ und N^{α}haaₒ einen Monomer-Aminoacyl-Aza-Beta-3-Rest oder eine Verkettung von Monomer-Aminoacyl-Aza-Beta-3-Resten analog zu den Aminoacylresten, die ursprünglich an derselben Position im Peptid oder parenten Protein vorhanden waren, von denen die Hybridpeptide ausgegangen sind, wobei die die Aminoacyl-Aza-Beta-3-Monomere den Formeln (A), (B) oder (C), die in Anspruch 1 angeführt sind, entsprechen, je nachdem, ob sie sich an der N-terminalen, C-terminalen Position oder in der Kette der Hybridpeptide befinden, und bei denen R₁ mit seitlichen Kette der ursprünglichen Aminosäure des Peptids oder des parenten Proteins, dem die Aminoacyl-Aza-Beta-3-Monomere entsprechen, identisch ist,
- l, m, n, o und p Null oder eine ganze Zahl zwischen 1 und 20 darstellen, vorbehaltlich dessen, dass mindestens m oder o ungleich Null ist, und dass die Mindestzahl von Resten in den Hybridpeptiden mit der Formel (I) gleich 4 ist und mindestens 1, n oder p ungleich Null ist.

7. Hybridpeptide nach Anspruch 5 oder 6 mit folgenden Formeln:
- SEQ ID NO : 2 (oder Peptid E):
⁸⁸H₂N-Tyr-Ala-Leu-Lys-Arg-Gln-Gly-Arg-Thr-**N^{α}-hLeu**-Tyr-Gly-OH⁹⁹
SEQ ID NO:3 (oder Peptid C):
⁸⁸H₂N-Tyr-Ala-**N^{α}-hLeu**-Lys-Arg-Gln-Gly-Arg-Thr-Leu-Tyr-Gly-OH⁹⁹
SEQ ID NO:4 (oder Peptid A):
⁸⁸H₂N-Tyr-**N^{α}-hAla-**Leu-Lys-Arg-Gln-Gly-Arg-Thr-Leu-Tyr-Gly-OH⁹⁹
- SEQ ID NO : 5 (oder Peptid B):
⁸⁸H₂N-Tyr-**N^{α}-hAla-N^{α}-hLeu**-Lys-Arg-Gln-Gly-Arg-Thr-Leu-Tyr-Gly-OH⁹⁹
- SEQ ID NO : 6 (oder Peptid D):
⁸⁸H₂N-Tyr-Ala-Leu-**N^{α}-hLys**-Arg-Gln-Gly-Arg-Thr-Leu-Tyr-Gly-OH⁹⁹
- SEQ ID NO : 7 (oder Peptid G) :
⁸⁸H₂N-Tyr-Ala-Leu-Lys-Arg-Gln-Gly-Arg-Thr-**N^{α}-hLeu-N^{α}-hTyr**-Gly-OH⁹⁹
- SEQ ID NO : 8 :
⁸⁸H₂N-Tyr-Ala-Leu-Lys-Arg-Gln-**N^{α}-hGly**-Arg-Thr-Leu-Tyr-Gly-OH⁹⁹
- SEQ ID NO : 9 :
⁸⁸H₂N-Tyr-Ala-Leu-Lys-Arg-Gln-Gly-**N^{α}-hArg**-Thr-Leu-Tyr-Gly-OH⁹⁹
- SEQ ID NO : 10 :
⁸⁸H₂N-Tyr-Ala-Leu-Lys-**N^{α}**-**hArg**-Gln-Gly-Arg-Thr-Leu-Tyr-Gly-OH⁹⁹
- SEQ ID NO : 11 :
⁸⁸H₂N-Tyr-Ala-Leu-Lys-Arg-Gln-Gly-Arg-Thr-Leu-**N^{α}-hTyr**-Gly-OH⁹⁹
- SEQ ID NO : 12 (oder Peptid F):
⁸⁸H₂N-**N^{α}-hTyr**-Ala-Leu-Lys-Arg-Gln-Gly-Arg-Thr-Leu-Tyr-Gly-OH⁹⁹
- SEQ ID NO : 13 (oder Peptid H) :
⁸⁸H₂N-Tyr-Ala-Leu-Lys-Arg-Gln-Gly-Arg-Thr-Leu-Tyr-**N^{α}-hGly**-OH⁹⁹
- SEQ ID NO : 14 (oder Peptid I) :
⁸⁸H₂N-Tyr-Ala-Leu-Lys-Arg-Gln-Gly-Arg-Thr-**N^{α}-hLeu-N^{α}-hTyr-N^{α}-hGly**-OH⁹⁹

8. Hybridpeptide nach einem der Ansprüche 5 bis 7 mit folgenden Formeln:
- **SEQ ID NO:2** (oder Peptid E):
⁸⁸H₂N-Tyr-Ala-Leu-Lys-Arg-Gln-Gly-Arg-Thr-**N^{α}-hLeu**-Tyr-Gly-OH⁹⁹
- **SEQ ID NO:7** (oder Peptid G) :
⁸⁸H₂N-Tyr-Ala-Leu-Lys-Arg-Gln-Gly-Arg-Thr-**N^{α}-hLea-N^{α}-hTyr**-Gly-OH⁹⁹

9. Monoklonale und polyklonale Hybridpeptid-Antikörper, wie durch Immunisierung eines Tiers mit mindestens einem in einem der Ansprüche 1 bis 8 definierten Hybridpeptid erhalten, wobei die Antikörper einen Komplex mit diesen Hybridpeptiden und/oder mit den parenten Peptiden oder Proteinen, die diesen letztgenannten entsprechen, bilden können, **dadurch gekennzeichnet, dass** sie das parente Peptid oder das parente Protein mit einer Affinität mindestens gleich jener erkennen, die von den Antikörpern gegen das parente Peptid oder das parente Protein gegenüber dem parenten Peptid oder dem parenten Protein gezeigt wird.

10. Idiotyp-Antikörper, die einen Komplex mit den Antikörpern nach Anspruch 9 bilden können, wie durch Immunisierung eines Tiers mit den Antikörpern nach Anspruch 9 erhalten.

11. Komplex zwischen einem Hybridpeptid, wie in einem der Ansprüche 1 bis 8 definiert, und einem Element des größeren Histokompatibilitätskomplexes (auch als MHC-Hybrid-Komplex bezeichnet) und eventuell einem T-Zellen-Rezeptor (oder auch als MHC-Hybrid-Rezeptor-T-Komplex bezeichnet).

12. Komplex zwischen einem Hybridpeptid, wie in einem der Ansprüche 1 bis 8 definiert, und einem T-Zellen-Rezeptor.

13. In-Vitro-Diagnosemethode von Pathologien, die mit dem Vorhandensein eines exogenen oder endogenen Proteins im Organismus eines Patienten zusammenhängen, das direkt oder indirekt am Auftreten und/oder an der Entwicklung dieser Pathologien beteiligt sein kann, **dadurch gekennzeichnet, dass** sie umfasst:
- die Herstellung eines Kontakts zwischen einer biologischen Probe, die von einem Patienten kommt, der Träger von gegen das Protein gerichteten Antikörpern sein kann, und einem Hybridpeptid, wie in einem der Ansprüche 1 bis 8, wobei das Hybridpeptid von der Gesamtheit oder einem Teil des endogenen oder exogenen Proteins oder von einem Peptid stammt, das durch Antikörper, die selbst das exogene oder endogene Protein erkennen können, erkannt werden kann,
unter Bedingungen, die die Reaktion zwischen den gegen das Protein gerichteten Antikörpern, die in der biologischen Probe vorhanden sein können, und dem oben erwähnten Hybridpeptid ermöglichen,
- die In-Vitro-Erfassung des Antigen-/Antikörper-Komplexes, der aus dem vorhergehenden Schritt gebildet werden kann, oder
- die In-Vitro-Erfassung von Antikörpern, die beim Patienten zirkulieren, durch einen Kompetitionstest unter Verwendung eines Hybrid-Antikörpers.

14. In-Vitro-Diagnosemethode von Pathologien, die mit dem Vorhandensein eines exogenen oder endogenen Proteins im Organismus eines Patienten zusammenhängen, das direkt oder indirekt am Auftreten und/oder an der Entwicklung dieser Pathologien beteiligt sein kann, wobei die Methode **dadurch gekennzeichnet ist, dass** sie umfasst:
- die Herstellung eines Kontakts zwischen einer biologischen Probe, die von einem Patienten kommt, der Träger des Proteins ist, mit mindestens einem der Antikörper nach Anspruch 9, wobei die Antikörper vorzugsweise gegen ein Hybridpeptid gerichtet sind, das von der Gesamtheit oder einem Teil des endogenen oder exogenen Proteins stammt,
unter Bedingungen, die die Reaktion zwischen dem Protein, das in der biologischen Probe vorhanden sein kann, und den Antikörpern, die gegen das oben erwähnte Hybridpeptid gerichtet sind, ermöglichen,
- die In-Vitro-Erfassung des Antigen-/Antikörper-Komplexes, der aus dem vorhergehenden Schritt gebildet werden kann, oder
- die In-Vitro-Erfassung von Antigenen, die beim Patienten zirkulieren, durch einen Kompetitionstest unter Verwendung eines Hybrid-Peptids nach einem der Ansprüche 1 bis 8.

15. Zubehör oder Kasten für en Einsatz von In-Vitro-Diagnosemethoden nach Anspruch 13 oder 14, umfassend:
- ein Hybridpeptid, das von der Gesamtheit oder einem Teil des endogenen oder exogenen - Proteins stammt oder einem Peptid entspricht, das von Antikörpern, die selbst das exogene oder endogene Protein erkennen, erkannt werden kann, oder Antikörper nach Anspruch 9, die gegen dieses Hybridpeptid gerichtet sind;
- Reagenzien, um ein Medium für die Bildung einer immunologischen Reaktion bereit zu machen;
- Reagenzien, die es ermöglichen, den Antigen/Antikörperkomplex zu erfassen, der nach der immunologischen Reaktion erzeugt wurde, wobei die Reagenzien eventuell einen Marker enthalten oder ihrerseits von einem markierten Reagens erkannt werden können, insbesondere wenn das oben erwähnte Hybridpeptid oder die Hybrid-Antikörper nicht markiert sind.

16. Pharmazeutische Zusammensetzung, insbesondere Impfstoff, **dadurch gekennzeichnet, dass** sie ein Hybridpeptid, wie in einem der Ansprüche 1 bis 8 definiert, oder ein Anti-Idiotyp nach Anspruch 10 in Verbindung mit einem physiologisch annehmbaren Träger oder nicht umfasst.

17. Pharmazeutische Zusammensetzung, **dadurch gekennzeichnet, dass** sie ein Hybridpeptid, wie in einem der Ansprüche 1 bis 8 definiert, oder einen Idiotyp-Antikörper nach Anspruch 10 umfasst, verbunden mit einem Trägermolekül proteinhaltiger Art oder nicht, das in vivo die Erzeugung von Antikörpern, die das exogene oder endogene Protein, das für die Pathologie verantwortlich ist, neutralisieren, oder in vivo eine zytotoxische oder Hilfszellimmunantwort herbeiführen kann.

18. Pharmazeutische Zusammensetzung, **dadurch gekennzeichnet, dass** sie Antikörper nach Anspruch 9 in Verbindung mit einem physiologisch annehmbaren Träger umfasst.


### LISTE DE SÉQUENCES

<110> CENTRE NATIONAL DE LA RECHERCHE SCIENTIFIQUE
<120> ANALOGUES PEPTIDIQUES COMPRENANT AU MOINS UN RESIDU AZA-β3-AMINOACYLE, ET LEURS UTILISATIONS, NOTAMMENT EN THERAPIE
<130> WOB 03 AQ CNR AZA3
<150> FR 03/06992
   <151> 2003-06-11
<160> 27
<170> Patent In version 3.1
<210> 1
   <211> 12
   <212> PRT
   <213> Homo sapiens
<400> 1
   Tyr Ala Leu Lys Arg Gln Gly Arg Thr Leu Tyr Gly
   1               5                   10
<210> 2
   <211> 12
   <212> PRT
   <213> Séquence artificielle
<220>
   <223> Peptide hybride
<220>
   <221> MISC_FEATURE
   <222> (10). .(10)
   <223> Nalpha-hLeucine
<400> 2
   Tyr Ala Leu Lys Arg Gln Gly Arg Thr Xaa Tyr Gly
   1               5                   10
<210> 3
   <211> 12
   <212> PRT
   <213> Séquence artificielle
<220>
   <223> Peptide hybride
<220>
   <221> MISC_FEATURE
   <222> (3) .. (3)
   <223> Nalpha-hLeucine
<400> 3
   Tyr Ala Xaa Lys Arg Gln Gly Arg Thr Leu Tyr Gly
   1 5 10
<210> 4
   <211> 12
   <212> PRT
   <213> Séquence artificielle
<220>
   <223> Peptide hybride
<220>
   <221> MISC_FEATURE
   <222> (2).. (2)
   <223> Nalpha-hAlanine
<400> 4
   Tyr Xaa Leu Lys Arg Gln Gly Arg Thr Leu Tyr Gly
   1 5 10
<210> 5
   <211> 12
   <212> PRT
   <213> Séquence artificielle
<220>
   <223> Peptide hybride
<220>
   <221> MISC_FEATURE
   <222> (2)..(2)
   <223> Nalpha-hAlanine
<220>
   <221> MISC_FEATURE
   <222> (3)..(3)
   <223> Nalpha-hLeucine
<400> 5
   Tyr Xaa Xaa Lys Arg Gln Gly Arg Thr Leu Tyr Gly
   1 5 10
<210> 6
   <211> 12
   <212> PRT
   <213> Séquence artificielle
<220>
   <223> Peptide hybride
<220>
   <221> MISC_FEATURE
   <222> (4) .. (4)
   <223> Nalpha-hLysine
<400> 6
   Tyr Ala Leu Xaa Arg Gln Gly Arg Thr Leu Tyr Gly
   1 5 10
<210> 7
   <211> 12
   <212> PRT
   <213> Séquence artificielle
<220>
   <223> Peptide hybride
<220>
   <221> MISC_FEATURE
   <222> (10)..(10)
   <223> Nalpha-hLeucine
<220>
   <221> MISC_FEATURE
   <222> (11)..(11)
   <223> Nalpha-hTyrosine
<400> 7
   Tyr Ala Leu Lys Arg Gln Gly Arg Thr Xaa Xaa Gly
   1 5 10
<210> 8
   <211> 12
   <212> PRT
   <213> Séquence artificielle
<220>
   <223> Peptide hybride
<220>
   <221> MISC_FEATURE
   <222> (7) .. (7)
   <223> Nalpha-hGlycine
<400> 8
   Tyr Ala Leu Lys Arg Gln Xaa Arg Thr Leu Tyr Gly
   1 5 10
<210> 9
   <211> 12
   <212> PRT
   <213> Séquence artificielle
<220>
   <223> Peptide hybride
<220>
   <221> MISC_FEATURE
   <222> (8) .. (8)
   <223> Nalpha-hArginine
<400> 9
   Tyr Ala Leu Lys Arg Gln Gly Xaa Thr Leu Tyr Gly
   1 5 10
<210> 10
   <211> 12
   <212> PRT
   <213> Séquence artificielle
<220>
   <223> Peptide hybride
<220>
   <221> MISC_FEATURE
   <222> (5)..(5)
   <223> Nalpha-hArginine
<400> 10
   Tyr Ala Leu Lys Xaa Gln Gly Arg Thr Leu Tyr Gly
   1 5 10
<210> 11
   <211> 12
   <212> PRT
   <213> Séquence artificielle
<220>
   <223> Peptide hybride
<220>
   <221> MISC_FEATURE
   <222> (11)..(11)
   <223> Nalpha-hTyrosine
<400> 11
   Tyr Ala Leu Lys Arg Gln Gly Arg Thr Leu Xaa Gly
   1 5 10
<210> 12
   <211> 12
   <212> PRT
   <213> Séquence artificielle
<220>
   <223> peptide hybride
<220>
   <221> MISC_FEATURE
   <222> (1) .. (1)
   <223> Nalpha-hTyrosine
<400> 12
   Xaa Ala Leu Lys Arg Gln Gly Arg Thr Leu Tyr Gly
   1 5 10
<210> 13
   <211> 12
   <212> PRT
   <213> Séquence artificielle
<220>
   <223> peptide hybride
<220>
   <221> MISC_FEATURE
   <222> (12)..(12)
   <223> Nalpha-hGlycine
<400> 13
   Tyr Ala Leu Lys Arg Gln Gly Arg Thr Leu Tyr Xaa
   1               5                   10
<210> 14
   <211> 12
   <212> PRT
   <213> Séquence artificielle
<220>
   <223> peptide hybride
<220>
   <221> MISC_FEATURE
   <222> (10)..(10)
   <223> Nalpha-hLeucine
<220>
   <221> MISC_FEATURE
   <222> (11)..(11)
   <223> Nalpha-hTyrosine
<220>
   <221> MISC_FEATURE
   <222> (12)..(12)
   <223> Nalpha-hGlycine
<400> 14
   Tyr Ala Leu Lys Arg Gln Gly Arg Thr Xaa Xaa Xaa
   1               5                   10
<210> 15
   <211> 13
   <212> PRT
   <213> Homo sapiens
   <400> 15
   Pro Lys Tyr Val Lys Gln Asn Thr Leu Lys Leu Ala Thr
   1               5                   10